(19) Europäisches Patentamt
European Patent Office
Office européen des brevets

(11) EP 4 516 287 A1

(12) EUROPEAN PATENT APPLICATION
published in accordance with Art. 153(4) EPC

(43) Date of publication:
05.03.2025 Bulletin 2025/10

(51) International Patent Classification (IPC):
A61K 9/00 (2006.01)     A61K 47/42 (2017.01)
A61P 25/00 (2006.01)    A61P 25/28 (2006.01)
A61P 37/00 (2006.01)    C07K 16/28 (2006.01)
A61K 39/00 (2006.01)

(21) Application number: 23792130.9

(22) Date of filing: 17.04.2023

(52) Cooperative Patent Classification (CPC):
A61K 9/00; A61K 38/47; A61K 39/00; A61K 47/42;
A61P 25/00; A61P 25/28; A61P 37/00; C07K 16/28

(86) International application number:
PCT/KR2023/005185

(87) International publication number:
WO 2023/204554 (26.10.2023 Gazette 2023/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.04.2022  KR 20220049046

(71) Applicant: Altos Biologies Inc.
Daejeon 34054 (KR)

(72) Inventors:
• LIM, Hyung Kwon
Daejeon 34054 (KR)
• JI, Hyi Jeong
Daejeon 34054 (KR)
• KIM, Kyuwan
Daejeon 34054 (KR)
• PARK, Soon Jae
Daejeon 34054 (KR)

(74) Representative: Icosa
83 avenue Denfert-Rochereau
75014 Paris (FR)

Remarks:
The complete document including Reference
Table(s) and the Sequence Listing(s) can be
downloaded from the EPO website

(54) PHARMACEUTICAL COMPOSITION COMPRISING OCRELIZUMAB AND USE THEREOF

(57) The present invention relates to a pharmaceutical composition for subcutaneous injection for the prevention or treatment of multiple sclerosis comprising ocrelizumab; and hyaluronidase, and administration dosage regimens thereof. Through the present invention, there is provided a novel drug administration method for the treatment of multiple sclerosis using CD20 antibody by reducing the side effects caused by intravenous injection, exhibiting efficacy equal to or higher than that of intravenous injection, and improving patient convenience.

[FIG. 6]

**Description**

**[Technical Field]**

The present invention relates to a pharmaceutical composition including ocrelizumab as an active ingredient and a method for treating multiple sclerosis using the same.

**[Background Art]**

Multiple sclerosis is a chronic inflammatory and demyelinating disease in which the myelin sheath composed of myelin and glycoprotein, which acts as an insulator surrounding the axon of nerve cells, is damaged and shed, and is an autoimmune disease that causes central nerve degeneration. Multiple sclerosis is characterized by accompanying symptoms such as dysesthesia due to sensory neuropathy, movement disorders (partial paralysis or quadriplegia), optic neuritis, *etc.*

Relapsing-remitting multiple sclerosis is a form in which relapse and recovery repeatedly occur after the onset of multiple sclerosis, and the lesions do not completely recover and remain disabled, and as the disease progresses, it gets worse, reaching a state that is difficult to recover from. Most patients with multiple sclerosis have relapsing-remitting sclerosis (about 85% to 90%), and primary progressive multiple sclerosis, in which the disease progresses progressively from the beginning without relapse, accounts for about 10% to 15% of the total number of patients. Another form of multiple sclerosis is secondary progressive multiple sclerosis, in which the disease progressively progresses as a chronic degenerative disease without obvious relapse after the first relapse and is rarely diagnosed.

A potential etiology of multiple sclerosis is the role of CD4+ Th1 T cells in the inflammatory responses. It is known that *IFN-beta* and glatiramer acetate, which are therapeutic agents that have been developed based on this mechanism, reduce the accumulation or aggravation of disability, but it cannot completely prevent relapse of the disease (KR 2013-0099228 A).

Recently, the importance of B cells in the pathology of multiple sclerosis has been reported, which is characterized by patients with early-stage multiple sclerosis and infiltration of inflammatory cells into active lesions. Infiltration of circulating B cells is observed to be much higher in multiple sclerosis than in other inflammatory diseases of the central nervous system, and in particular, CD20+ B cells have been found to be involved in the inflammatory responses as they are centered around blood vessels (Machado-Santos J *et al.,* 2018; Magliozzi R *et al.,* 2007).

CD20 is a non-glycosylated 33 kDa to 37 kDa phosphoprotein that penetrates the cell membrane and forms a tetramer coupled with lipid rafts, which are lipid complexes present in the cell membrane. CD20 is expressed in more than 90% of B cells derived from circulating blood or lymphoid organs. When classified by developmental stage, CD20 is highly expressed from pre-B cells, the precursor stage of B cell differentiation, to mature B cells and memory B cells. Interestingly, CD20 expression is also observed in B-cell lymphomas, leukemias, some myeloma, thymoma, and T cells of Hodgkin's disease. In particular, in the case of non-Hodgkin's disease, although it is expressed in more than 90% of B cells, it is not found in pro-B cells, normal plasma cells, or other normal tissues. CD20 has been reported to be involved in cell cycle initiation, plasma cell differentiation, T cell activity, and calcium ion channel activity (Teddler *et al.* 1990).

Based on the findings that CD20 is highly expressed in B-cell lymphoma and autoimmune diseases, CD20 has been recognized and studied as an important therapeutic target, and the development of anti-CD20 monoclonal antibodies, which are drugs that deplete CD20+ B cells, has been accelerated (Edwards et al., Biochem Scoc. Trans. 30:824-828 (2002). Anti-CD20 antibodies currently approved for the treatment of multiple sclerosis include ocrelizumab in the form of intravenous injection and ofatumumab in the form of subcutaneous injection. In addition, rituximab is being prescribed off-label, and there are other CD20 targeted antibodies such as Obinutuzumab and Ublituximab, *etc.* Anti-CD20 monoclonal antibody is a therapeutic agent that selectively depletes B cells and is effectively applied to the treatment of multiple sclerosis patients due to its high efficacy and drug safety (Hauser *et al.,* 2008; Montalban *et al.,* 2017; Hauser *et al., 2020).*

Under the circumstances where the etiology of multiple sclerosis is not clear and there is no perfect treatment, the development of anti-CD20 antibody therapeutics has received significant attention and is still underway. Among these, Ocrelizumab is a humanized anti-CD20 antibody that has superior efficacy compared to existing compound therapeutics and has been successfully applied in the market of multiple sclerosis treatment (Klein *et al.,* 2013; Gelfand *et al.,* 2017).

However, safety issues have been raised for anti-CD20 antibody therapeutics due to acute allergic or IgE-linked hypersensitivity side effects that can appear within 24 hours after the initial injection (Hauser *et al.,* 2017; Ostergaard *et al.,* 2010). In addition, problems caused by intravenous injection have been raised, and side effects due to the large volume of injection solution, long injection time, infection, blood vessel damage, or thrombosis, *etc.* have also been raised as problems. Further, in the case of elderly or pediatric patients and patients with circulation problems caused by intravenous injection can be serious (Shire *et al.,* 2004; Roskos *et al.,* 2004).

In particular, a report of the European regulatory authority warns that there is a possibility of side effects caused by intravenous injection of anti-CD20 antibodies including ocrelizumab, and requires that the approved dosage regimens be used for intravenous infusion. Accordingly, careful preparation and observation are required before and after injection, and in case of adverse reactions related to injection, it is recommended to discontinue the injection and reduce the injection rate (EMA, Summary of Product Characteristics for Ocrevus 300 mg concentrate for solution for infusion).

In order to solve the side effects of the existing method of intravenous injection of CD20 antibody therapeutics and to increase patient convenience and effectiveness of drug treatment, the development of a method for administering antibody therapeutics in the form of subcutaneous injection along with the identification of the effective dosage regimens that can be applied to actual clinical practice are required as a major technical challenge to be solved.

In the present invention, according to the medical needs of developing a subcutaneous formulation of ocrelizumab, which is applied as a treatment for multiple sclerosis, the dosage regimens of the subcutaneous formulation are tested and selected.

## [Disclosure]

## [Technical Problem]

It is one object of the present invention to provide a pharmaceutical composition for preventing or treating multiple sclerosis, including ocrelizumab; and hyaluronidase, wherein, in the composition, ocrelizumab is subcutaneously administered to patients with multiple sclerosis at a dose of 40 mg to 1,200 mg.

It is another object of the present invention to provide a pharmaceutical composition for preventing or treating multiple sclerosis, including 250 mg to 750 mg of ocrelizumab; and hyaluronidase.

It is still another object of the present invention to provide a pharmaceutical composition for CD20 targeted therapy, including ocrelizumab; and hyaluronidase, wherein, in the composition, ocrelizumab is subcutaneously administered at a dose of 40 mg to 1,200 mg to patients targeted for CD20 targeted therapy.

It is yet another object of the present invention to provide a method for preventing or treating multiple sclerosis, including: subcutaneously administering 40 mg to 1,200 mg of ocrelizumab or a pharmaceutical composition including the same to a subject.

## [Technical Solution]

One aspect for implementing the present invention provides a pharmaceutical composition for preventing or treating multiple sclerosis, including ocrelizumab; and hyaluronidase.

In one embodiment, the pharmaceutical composition is a pharmaceutical composition for preventing or treating multiple sclerosis, including ocrelizumab; and hyaluronidase, wherein the ocrelizumab in the pharmaceutical composition is subcutaneously administered to patients with multiple sclerosis at a dose of 40 mg to 1,200 mg.

As the pharmaceutical composition according to the above-described embodiments, the pharmaceutical composition targets CD20.

As the pharmaceutical composition according to the above-described embodiments, the hyaluronidase is selected from the group consisting of a combination of human or animal-derived PH20 and variants thereof having enzymatic activity at neutral pH.

As the pharmaceutical composition according to any one of the above-described embodiments, the hyaluronidase may include an amino acid sequence of SEQ ID NO: 2, but is not limited thereto.

As the pharmaceutical composition according to any one of the above-described embodiments, the ocrelizumab is subcutaneously administered to patients with multiple sclerosis at a dose of 125 mg to 750 mg.

As the pharmaceutical composition according to any one of the above-described embodiments, the pharmaceutical composition includes 1,000 U/mL to 12,000 U/mL of hyaluronidase.

As the pharmaceutical composition according to any one of the above-described embodiments, the pharmaceutical composition further includes a buffer at pH 5.5±2.0, a stabilizer, a nonionic surfactant, an amino acid, and a combination thereof.

As the pharmaceutical composition according to any one of the above-described embodiments, the pharmaceutical composition is administered once every 2 to 28 weeks.

As the pharmaceutical composition according to any one of the above-described embodiments, the pharmaceutical composition exhibits efficacy equal to or higher than that of intravenous administration as well as improves infusion related reactions (IRR).

Another aspect for implementing the present invention provides a pharmaceutical composition for preventing or treating multiple sclerosis, including 250 mg to 750 mg of ocrelizumab; and hyaluronidase

In one embodiment, the pharmaceutical composition is subcutaneously administered once or more every 4 to 28 weeks.

Still another aspect for implementing the present invention provides a pharmaceutical composition for preventing or treating multiple sclerosis, including 375 mg to 750 mg of ocrelizumab; and hyaluronidase.

In one embodiment, the pharmaceutical composition is subcutaneously administered once or more every 8 to 28 weeks.

Yet another aspect for implementing the present invention provides a pharmaceutical composition for CD20 targeted therapy, including ocrelizumab; and hyaluronidase, wherein, in the composition, ocrelizumab is subcutaneously administered at a dose of 40 mg to 1,200 mg to patients targeted for CD20 targeted therapy.

Even another aspect for implementing the present invention provides a method for preventing or treating multiple sclerosis, including: subcutaneously administering 40 mg to 1,200 mg of ocrelizumab or a pharmaceutical composition including the same to a subject.

In one embodiment, the administration is performed once every 2 to 24 weeks.

In one embodiment, the method further includes administering hyaluronidase, and in particular, the hyaluronidase is administered simultaneously, sequentially, or in reverse order with ocrelizumab

Further another aspect for implementing the present invention provides a formulation for subcutaneous administration for the prevention or treatment of multiple sclerosis, including 125 mg to 750 mg of ocrelizumab; and hyaluronidase.

In one embodiment, the administration is performed once every 2 to 24 weeks.

**[Advantageous Effects]**

The pharmaceutical composition including ocrelizumab according to the present invention can be subcutaneously administered in appropriate dosage regimens, and thus can be applied to the treatment of multiple sclerosis as well as reduce side effects.

**[Brief Description of Drawings]**

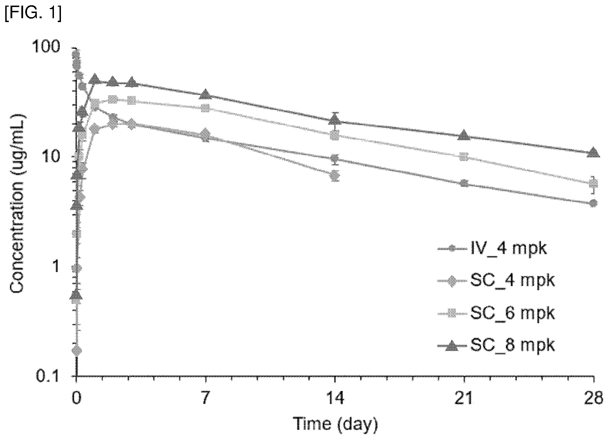

FIG. 1 is the result of confirming the pharmacokinetic properties of ocrelizumab when formulations of subcutaneous injection (SC) containing ocrelizumab and hyaluronidase according to the present invention were each subcutaneously injected at doses of 4 mg/kg, 6 mg/kg, and 8 mg/kg and intravenously injected (IV) at 4 mg/kg.

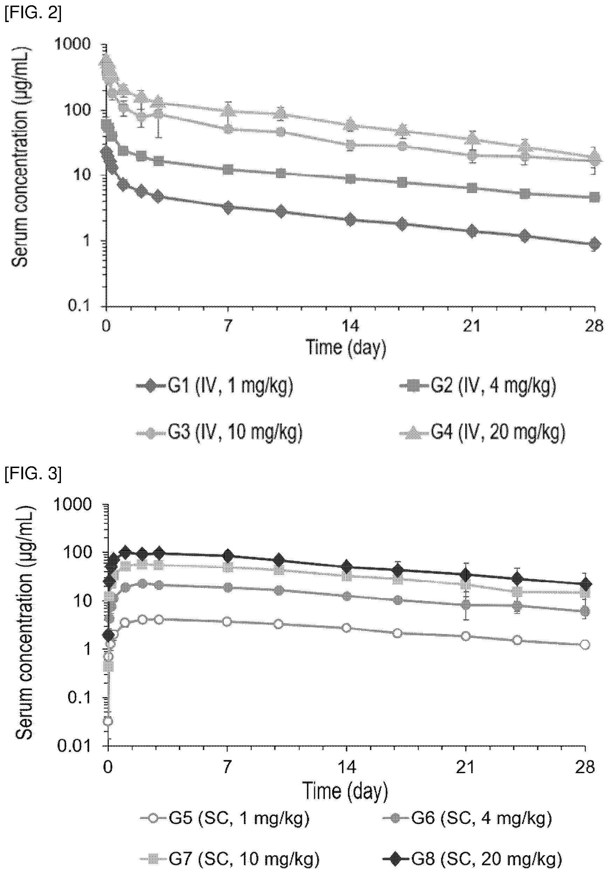

FIG. 2 is the result of confirming the pharmacokinetic properties of ocrelizumab when formulations of intravenous injection (IV) containing ocrelizumab according to the present invention were administered at doses of 1 mg/kg, 4 mg/kg, 10 mg/kg, 20 mg/kg by expanding the range of drug concentration thereof.

FIG. 3 is the result of confirming the pharmacokinetic properties of ocrelizumab when formulations of subcutaneous injection (SC) containing ocrelizumab and hyaluronidase according to the present invention were injected at doses of 1 mg/kg, 4 mg/kg, 10 mg/kg, and 20 mg/kg by expending the range of drug concentration thereof.

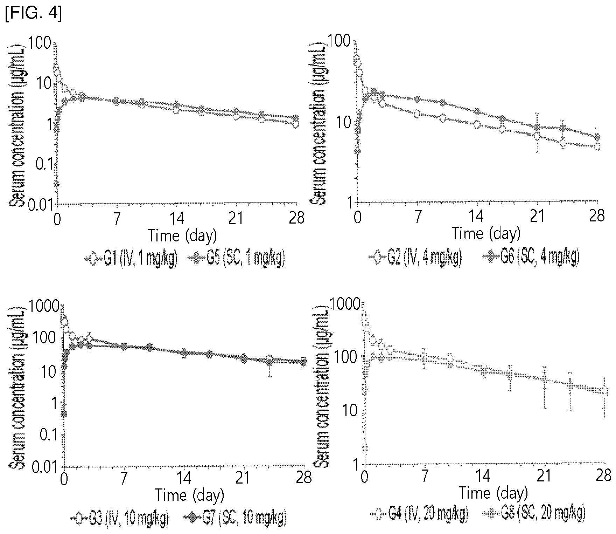

FIG. 4 is the result of pairing the pharmacokinetics for the same dose of ocrelizumab observed when the formulations of ocrelizumab intravenous injection (G1-G4) and the formulations of subcutaneous injection (G5-G8) including ocrelizumab and hyaluronidase according to the present invention were each injected at doses of 1 mg, 4 mg, 10 mg, and 20 mg doses.

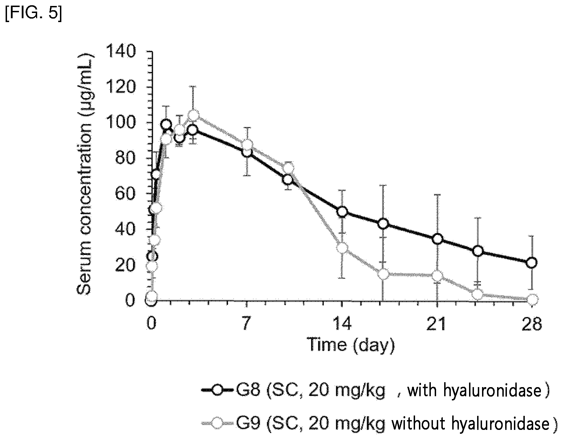

FIG. 5 is the result of confirming the pharmacokinetic properties when the formulations of ocrelizumab subcutaneous injection according to the present invention were subcutaneously injected with or without hyaluronidase at a dose of 20 mg/kg.

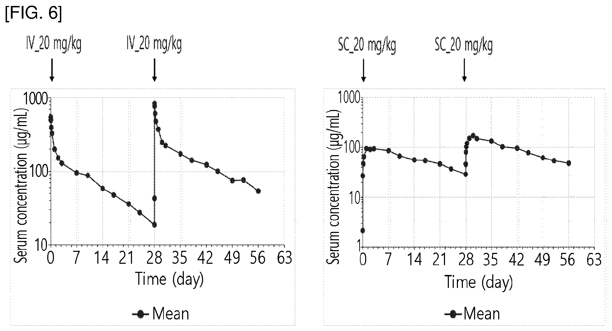

FIG. 6 shows graphs showing the pharmacokinetics when the formulations of the ocrelizumab intravenous injection and the formulations of subcutaneous injection including ocrelizumab and hyaluronidase according to the present invention were repeatedly administered at a dose of 20 mg/kg.

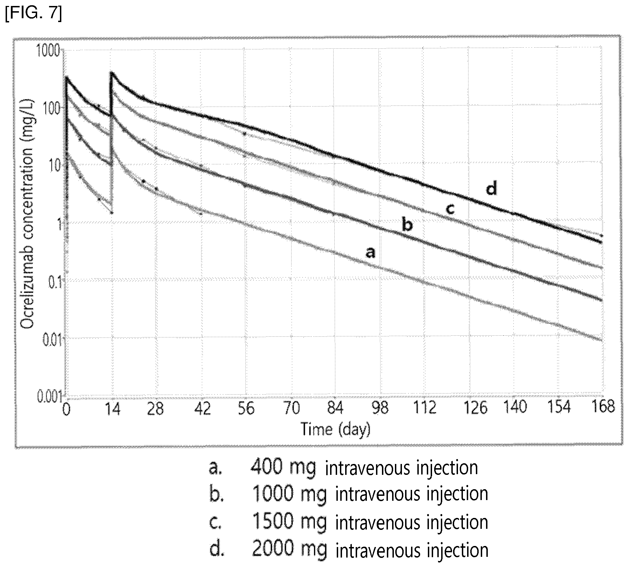

FIG. 7 is a diagram comparing the pharmacokinetics model established under the assumption that 400 mg, 1,000 mg, 1,500 mg, and 2,000 mg of ocrelizumab are intravenously administered to the human body and the pharmacokinetics measured during actual intravenous injection of ocrelizumab.

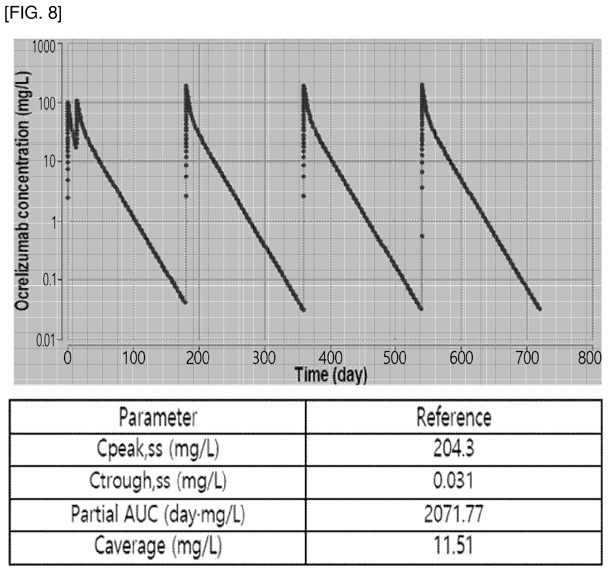

FIG. 8 is a model predicting pharmacokinetics under the assumption that 600 mg of ocrelizumab is injected 4 times over 2 years at 6-month intervals and the initial injection is carried out in a way that 300 mg of ocrelizumab is administered twice at 2-week intervals, as the standard dosage regimens of ocrelizumab applied to the treatment of multiple sclerosis.

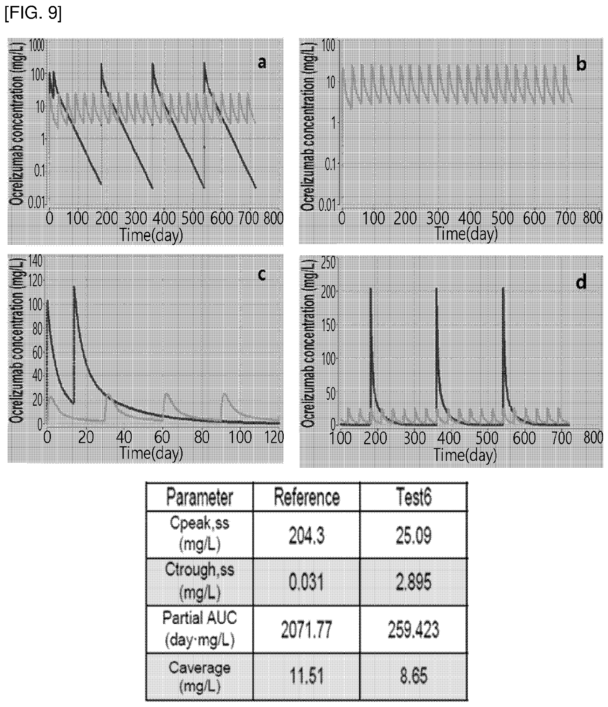

FIG. 9 shows graphs comparing the pharmacokinetic simulations when 125 mg of ocrelizumab containing hyaluronidase was subcutaneously administered 24 times at 1-month intervals to the human body and the pharmacokinetic simulations when 600 mg of ocrelizumab was intravenously administered 4 times at 6-month intervals in actual clinical practice. Specifically, each graph indicates the following: (a) Overlap comparison of simulations of standard intravenous injection at a dose of 600 mg (bold) and simulations of subcutaneous injection at a dose of 125 mg administered at 1-month intervals (light), (b) Simulations of subcutaneous injection at a dose of 125 mg at 1-month intervals, (c) Overlap comparison of simulations of intravenous injection at a dose of 300 mg administered twice at 2-week intervals for the first 6 months (bold) and simulations of subcutaneous injection at a dose of 125 mg administered at 1-month interval (light), and (d) Overlap comparison of simulations of intravenous injection administered at a dose of 600 mg after 6 months (bold) and simulations of subcutaneous injection at a dose of 125 mg administered at 1-month intervals (yellow).

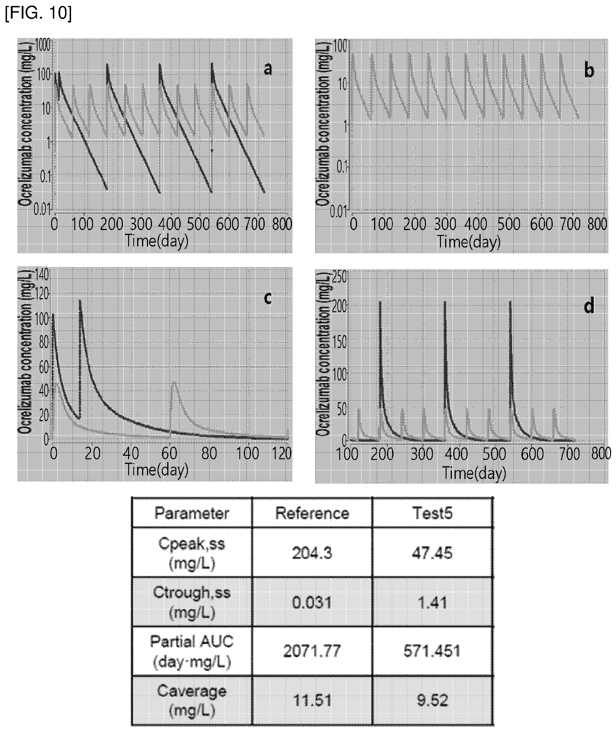

FIG. 10 shows graphs comparing the pharmacokinetic simulations when 250 mg of ocrelizumab containing hyaluronidase was subcutaneously administered 12 times at 2-month intervals to the human body and the pharmacokinetic simulations when 600 mg of ocrelizumab was intravenously administered 4 times at 6-month intervals in actual clinical practice. Specifically, each graph indicates the following: (a) Overlap comparison of simulations of standard intravenous injection at a dose of 600 mg (bold) and simulations of subcutaneous injection at a dose of 250 mg administered at 2-month intervals (light), (b) Simulations of subcutaneous injection at a dose of 250 mg at 2-month intervals, (c) Overlap comparison of simulations of intravenous injection at a dose of 300 mg administered twice at 2-week intervals for the first 6 months (bold) and simulations of subcutaneous injection at a dose of 250 mg administered at 2-month interval (light), and (d) Overlap comparison of simulations of intravenous injection administered at a dose of 600 mg after 6 months (bold) and simulations of subcutaneous injection at a dose of 250 mg administered at 2-month intervals (yellow).

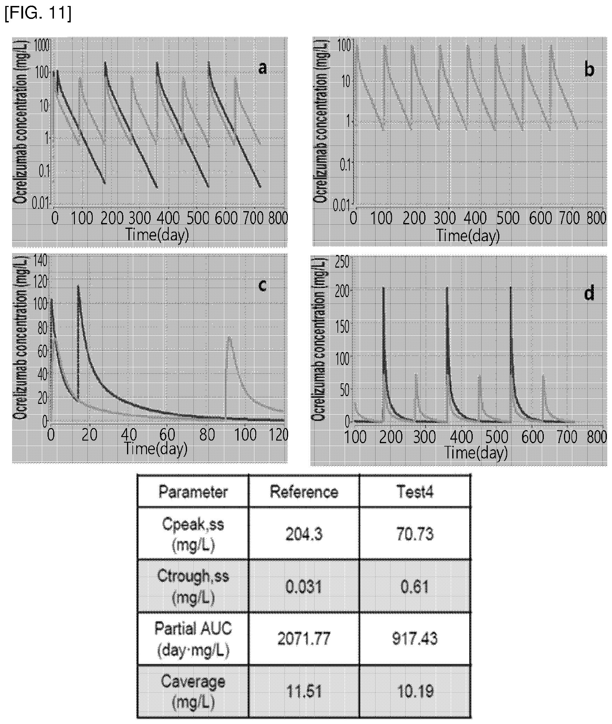

FIG. 11 shows graphs comparing the pharmacokinetic simulations when 375 mg of ocrelizumab containing hyaluronidase was subcutaneously administered 8 times at 3-month intervals to the human body and the pharmacokinetic simulations when 600 mg of ocrelizumab was intravenously administered 4 times at 6-month intervals in actual clinical practice. Specifically, each graph indicates the following: (a) Overlap comparison of simulations of standard intravenous injection at a dose of 600 mg (bold) and simulations of subcutaneous injection at a dose of 375 mg administered at 3-month intervals (light), (b) Simulations of subcutaneous injection at a dose of 375 mg at 3-month intervals, (c) Overlap comparison of simulations of intravenous injection at a dose of 300 mg administered twice at 2-week intervals for the first 6 months (bold) and simulations of subcutaneous injection at a dose of 375 mg administered at 3-month interval (light), and (d) Overlap comparison of simulations of intravenous injection administered at a dose of 600 mg after 6 months (bold) and simulations of subcutaneous injection at a dose of 375 mg administered at 3-month intervals (yellow).

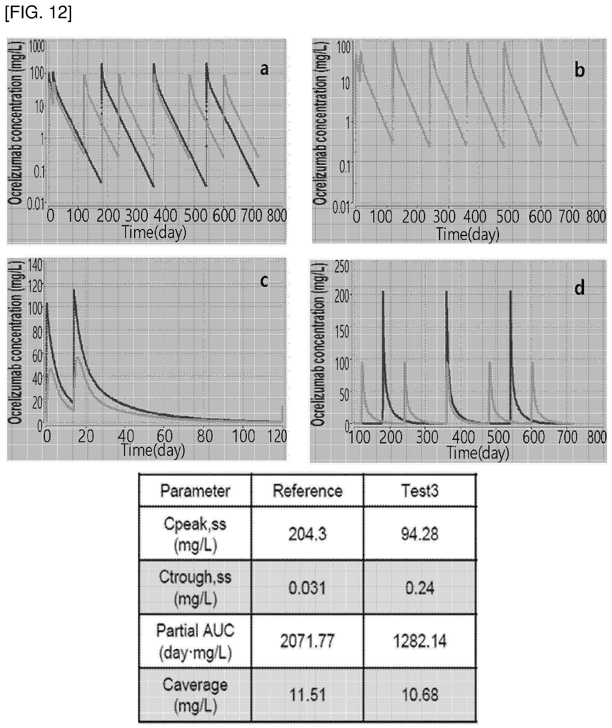

FIG. 12 shows graphs comparing the pharmacokinetic simulations when 500 mg of ocrelizumab containing hyaluronidase was subcutaneously administered 6 times at 4-month intervals to the human body, while the initial administration was carried out in a way that 250 mg of ocrelizumab was administered twice in a divided dose, and the pharmacokinetic simulations when 600 mg of ocrelizumab was intravenously administered 4 times at 6-month intervals in actual clinical practice. Specifically, each graph indicates the following: (a) Overlap comparison of simulations of standard intravenous injection at a dose of 600 mg (bold) and simulations of subcutaneous injection at a dose of 500 mg administered at 4-month intervals (light), (b) Simulations of subcutaneous injection at a dose of 500 mg at 4-month intervals, (c) Overlap comparison of simulations of intravenous injection at a dose of 300 mg administered twice at 2-week intervals as the initial administration (bold) and simulations of subcutaneous injection at a dose of 250 mg administered twice at 2-week interval as the initial administration (light), and (d) Overlap comparison of simulations of intravenous injection administered at a dose of 600 mg after 6 months (bold) and simulations of subcutaneous injection at a dose of 500 mg administered after 4 months (light).

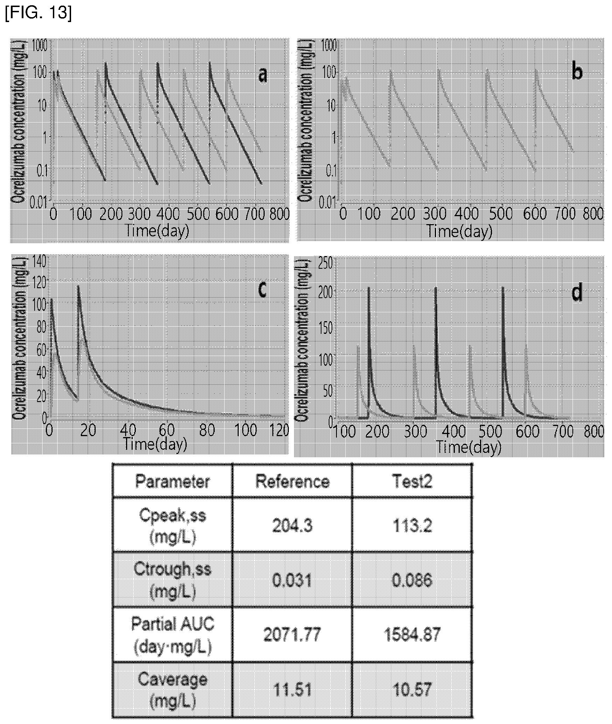

FIG. 13 shows graphs comparing the pharmacokinetic simulations when 600 mg of ocrelizumab containing hyaluronidase was subcutaneously administered 5 times at 5-month intervals to the human body, while the initial administration was carried out in a way that 300 mg of ocrelizumab was administered twice at 2-week intervals, and the pharmacokinetic simulations when 600 mg of ocrelizumab was intravenously administered 4 times at 6-month intervals in actual clinical practice. Specifically, each graph indicates the following: (a) Overlap comparison of simulations of standard intravenous injection at a dose of 600 mg (bold) and simulations of subcutaneous injection at a dose of 600 mg administered at 5-month intervals (light), (b) Simulations of subcutaneous injection at a dose of 600 mg at 5-month intervals, (c) Overlap comparison of simulations of intravenous injection at a dose of 300 mg administered twice at 2-week intervals as the initial administration (bold) and simulations of subcutaneous injection at a dose of 300 mg administered twice at 2-week interval as the initial administration (light), and (d) Overlap comparison of simulations of intravenous injection administered at a dose of 600 mg after 6 months (bold) and simulations of subcutaneous injection at a dose of 600 mg administered after 5 months (light).

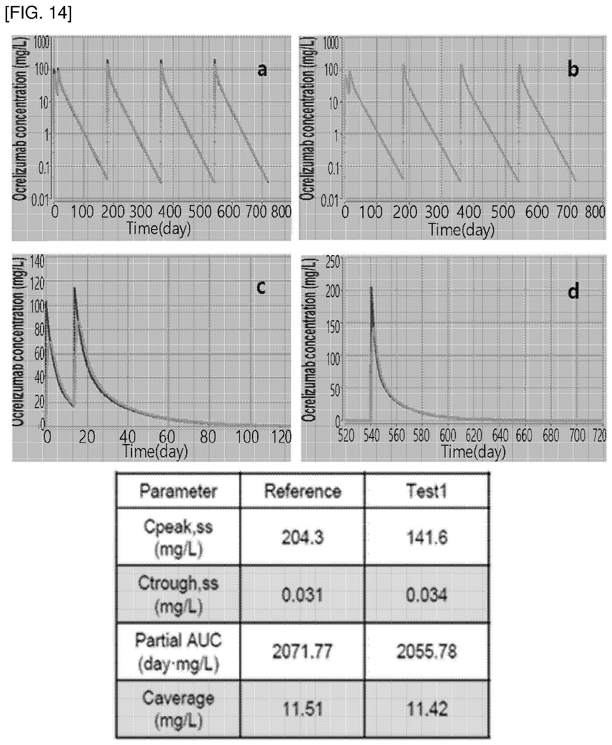

FIG. 14 shows graphs comparing the pharmacokinetic simulations when 750 mg of ocrelizumab containing hyaluronidase was subcutaneously administered 4 times at 6-month intervals to the human body, while the initial administration was carried out in a way that 375 mg of ocrelizumab was administered twice at 2-week intervals, and the pharmacokinetic simulations when 600 mg of ocrelizumab was intravenously administered 4 times at 6-month intervals in actual clinical practice. Specifically, each graph indicates the following: (a) Overlap comparison of simulations of standard intravenous injection at a dose of 600 mg (bold) and simulations of subcutaneous injection at a dose of 750 mg administered at 6-month intervals (light), (b) Simulations of subcutaneous injection at a dose of 750 mg at 6-month intervals, (c) Overlap comparison of simulations of intravenous injection at a dose of 300 mg administered twice at 2-week intervals as the initial administration (bold) and simulations of subcutaneous injection at a dose of 375 mg administered twice at 2-week interval as the initial administration (light), and (d) Overlap comparison of simulations of intravenous injection administered at a dose of 600 mg after 6 months (bold) and simulations of subcutaneous injection at a dose of 750 mg administered after 6 months (light).

[Detailed Description of Embodiments]

Hereinafter, the present invention will be described in detail.

Meanwhile, each description and embodiment disclosed herein can be applied to other descriptions and embodiments, respectively. That is, all combinations of various elements disclosed herein fall within the scope of the present invention. Further, the scope of the present invention is not limited by the specific description described below. Moreover, a number of papers and patent documents have been cited throughout the present specification. The content of the cited papers and patent documents is incorporated herein by reference in their entirety and the level of technical field to which the present invention belongs and the contents of the present invention will be described more clearly.

One aspect for implementing the present invention provides a pharmaceutical composition for preventing or treating multiple sclerosis, including ocrelizumab; and hyaluronidase, wherein the ocrelizumab in the pharmaceutical composition is administered to patients with multiple sclerosis at a dose of 40 mg to 1,200 mg.

In the treatment of multiple sclerosis, CD20 has been studied as an important therapeutic target, and the pharmaceutical composition of the present invention contains ocrelizumab, an anti-CD20 monoclonal antibody, which is a drug that depletes CD20+ B cells, thereby providing excellent effects in patients with multiple sclerosis. Although the effects of ocrelizumab on patients with multiple sclerosis have been studied, development of therapeutics for multiple sclerosis that can be applied in clinical trials is insignificant because conventional intravenous administration is accompanied by a number of side effects. The main side effect of anti-CD20 multiple sclerosis therapeutics compared to blinding known to date is infusion-related reaction (IRR). It has been reported that rituximab has IRR of 67.1% (23.1% for blinding) in Phase 2/3 of the OLYMPUS clinical trial and ocrelizumab has IRR of 39.9% (25.5% for blinding) in Phase 3 of the ORATORIO clinical trial (Cotchett et al. 2021)).

As described above, intravenous administration of ocrelizumab induces IRR grade variation, which is a problem, and thus it is necessary to identify new administration formulations and dosage regimen. Accordingly, the present invention has major technical features in that a formulation capable of subcutaneously administering ocrelizumab has been developed and at the same time identifies an appropriate dose so that it can exhibit the same or higher efficacy as intravenous administration.

In a specific embodiment of the present invention, the pharmaceutical composition of the present invention may be a stabilized pharmaceutical composition to maintain activity in a highly concentrated state of ocrelizumab such that it can be subcutaneously administered.

As used herein, "ocrelizumab (Ocrevus, Roche)" refers to a humanized anti-CD20 monoclonal antibody developed by Roche/Regeneron, it is prescribed in that the initial 300 mg dose is administered as intravenous injection, followed 2 weeks later by a second 300 mg infusion, and subsequent doses thereafter are administered as intravenous injection every 6 months. The injection solution is supplied with a dose of 300 mg in a disposable vial at a concentration of 30 mg/mL. For single initial dose, 300 mg is diluted in 250 mL of saline and injected, and thereafter, 600 mg is diluted in 500 mL of saline and injected. This product is an injectable liquid formulation composed of acetic acid (0.25 mg/mL), polysorbate 20 (0.2 mg/mL), chloroacetic acid trihydrate (2.14 mg/mL) and trehalose dihydrate (40 mg/mL), and the pH is adjusted to 5.3.

The pharmaceutical composition of the present invention includes hyaluronidase so that ocrelizumab can be safely subcutaneously administered.

As used herein, the term "hyaluronidase" refers to an enzyme that degrades hyaluronic acid in which N-acetyl-D-glucosamine and D-glucuronic acid are repeatedly linked multiple times. In general, the volume of the injection formulation through the subcutaneous route is limited to about 2 mL. If a patient requires multiple administration, the dose is divided into small units and injected into several skin surface areas on the body surface. When hyaluronidase is added to a therapeutic drug formulation and subcutaneously administered, hyaluronic acid distributed in the extracellular matrix is hydrolyzed to reduce the subcutaneous viscosity, thus increasing material permeability, and as a result, a high-dose drug can be easily delivered into the body. Therefore, in the present invention, it was found that the problem of the volume limitation of the dose can be overcome by co-administering hyaluronidase.

In the present invention, hyaluronidase is not limited to a specific form and specific dose as long as it can stabilize ocrelizumab to be subcutaneously administered.

For example, the hyaluronidase and ocrelizumab may be administered as single formulation or separate formulations. Specifically, when the hyaluronidase and ocrelizumab are administered as separate formulations, the hyaluronidase and ocrelizumab may be administered simultaneously, sequentially, or in reverse order. In a more specific example, when the hyaluronidase and ocrelizumab are administered as separate formulations, the formulation containing hyaluronidase may be administered first and the formulation containing ocrelizumab may be sequentially administered thereafter, but the order is not limited. In another more specific example, when the hyaluronidase and ocrelizumab are administered as separate formulations, the formulation containing ocrelizumab may be administered first, and the formulation containing hyaluronidase is sequentially administered thereafter, but the order is not limited thereto.

More specifically, the pharmaceutical composition including ocrelizumab and hyaluronidase of the present invention may be prepared as follows:

(i) ocrelizumab and (ii) hyaluronidase may be included as single formulation, or

(i) ocrelizumab and (ii) hyaluronidase may be prepared into separate formulations and included therein, but the formulation is not limited thereto.

If (i) ocrelizumab and (ii) hyaluronidase are prepared into separate formulations, they may be provided in the form of a kit, but the formulation is not limited thereto.

The concentration of the hyaluronidase in the pharmaceutical composition of the present invention may vary depending on the type of hyaluronidase used in the development of formulations. When the hyaluronidase is co-administered with ocrelizumab, a dose sufficient to increase the distribution and absorption of the antibody should be provided, and in this regard, the effective amount of hyaluronidase is 1,000 U/mL to 16,000 U/mL, which is converted to be about 0.01 mg to 0.15 mg in terms of protein content when the activity is calculated as 100,000 U/mg. More specifically, the dose of hyaluronidase may be 1,500 U/mL to 12,000 U/mL, and more specifically, the dose of hyaluronidase may be about 2,000 U/mL or 12,000 U/mL. The dose of hyaluronidase given above may be initially included in the drug formulation or may be prepared for simultaneous administration.

Specifically, the pharmaceutical composition of the present invention may include hyaluronidase in an amount of about 500 U/mL to about 12,000 U/mL, about 500 U/mL to about 5,000 U/mL, about 1,000 U/mL to about 5,000 U/mL, about 2,000 U/mL to about 5,000 U/mL, about 50 U/mL to about 2,000 U/mL, about 500 U/mL to about 2,000 U/mL, about 1,000 U/mL to about 2,000 U/mL in addition to ocrelizumab, but is not limited thereto. In one example, the pharmaceutical composition of the present invention may include 1,000 U/mL to 12,000 U/mL of hyaluronidase, but is not limited thereto.

The pharmaceutical composition of the present invention is prepared to have a formulation suitable for subcutaneous administration including ocrelizumab and hyaluronidase, and may be administered in dosage regimens that can obtain the same pharmacological effects as intravenous administration even during subcutaneous administration.

In the present invention, the hyaluronidase may be derived from animals or humans, and may be prepared based on recombinant DNA technology, but is not limited thereto.

There are six types of human hyaluronidase derived from human: Hya11, Hya12, Hya13, Hya14, HyalPS1, and PH20/SPAM1. Among them, hyaluronidase PH20, which is used in the development of formulations of subcutaneous injection is an enzyme (EC 3.2.1.35) that cleaves β-1,4 bonds between N-acetylglucosamine and glucuronic acid, which are sugars constituting hyaluronic acid. Human hyaluronidase PH20 has an optimal pH of 5.5, but exhibits some activity even at a pH of 7 to 8, whereas other human hyaluronidases, such as Hyal1, Hyal2, Hyal3, HYal4, *etc.,* have an optimal pH of 3 to 4 and have very weak activity at a pH of 7 to 8. The pH of subcutaneous areas in a human is about 7.4, which is substantially neutral, and thus, among various types of hyaluronidases, PH20 is widely applied in clinical use. Hyaluronidases such as Hyal1 may be mixed with drugs in excess amount or may be modified by genetic engineering and applied to the subcutaneous areas (pH 7.4) of human.

Additionally, the hyaluronidase may be PH20 derived from mammals such as cattle, sheep, pigs, *etc.* In addition, the hyaluronidase may be a hyaluronidase derived from a microorganism having activity at neutral pH, or may be a hyaluronidase obtained from bee venom or leech.

In the present invention, the hyaluronidase may be a wild-type PH20 having an amino acid sequence of SEQ ID NO: 1, but is not limited thereto. SEQ ID NO: 1 includes a signal sequence, and in a more specific embodiment, the wild-type PH20 may include a sequence including amino acids at positions 36 to 482 from the N-terminus of SEQ ID NO: 1, but is not limited thereto.

In a specific example, the hyaluronidase included in the pharmaceutical composition of the present invention may be one selected from the group consisting of a combination of human or animal-derived PH20 and variants thereof having enzymatic activity at neutral pH, but is not limited thereto. The PH20 variant may be a PH20 variant according to Korean Patent No. 10-2151388, and the entire specification of Korean Patent No. 10-2151388 may be incorporated herein by reference. The pharmaceutical composition according to the present invention includes practical examples of pharmaceutical compositions including human hyaluronidase PH20 and variants thereof.

The PH20 variant of the present invention may have the activity of wild-type PH20 (SEQ ID NOs: 1, and 36 to 482) and as well as have a certain level of homology, for example, homology or identity of 70% or more, 80% or more, 85% or more, 90% or more, 91% or more, 92% or more, 93% or more, 94% or more, 95% or more, 96% or more, 97% or more, 98% or more, or 99% or more, but is not limited thereto. Specifically, one or more amino acids in the wild-type PH20 sequence may include a modification selected from the group consisting of substitution, deletion, modification, addition, and a combination thereof.

Examples of the PH20 variant may include: one in which one or more amino acids at positions 341, 343, 349, 353, 354, 356, and 361 from the N-terminus of the wild-type PH20 are substituted; one in which a part of the N-terminus or C-terminus is deleted; or one in which 1 to 22 amino acids are substituted, but is not limited thereto. More specifically, the PH20 variant may include the amino acid sequence of SEQ ID NO: 2, and more specifically, may include a sequence including amino acids at positions 38 to 468 from the N-terminus of SEQ ID NO: 2, but is not limited thereto.

As used herein, the term "homology" or "identity" refers to a degree of similarity between two given amino acid sequences or nucleotide sequences, and may be expressed as a percentage.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

The terms homology and identity may often be used interchangeably with each other.

Whether any two nucleotide sequences or peptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of nucleotide sequences or peptide sequences may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (*i.e.,* nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" used herein indicates relatedness between sequences.

The pharmaceutical composition of the present invention may be administered to patients with multiple sclerosis in an amount of about 40 mg to about 1,200 mg, about 125 mg to 1,125 mg, about 125 mg to 750 mg, about 250 mg to 500 mg, about 250 mg to 750 mg, about 375 mg to 500 mg, about 375 mg to 1,125 mg, about 375 mg to 750 mg, about 500 mg to 1,125 mg, or about 500 mg to 750 mg of ocrelizumab in a single or divided doses. For each administration, the composition may be administered in divided doses.

As used herein, the term "about" refers to a range which includes all of $\pm0.5$, $\pm0.4$, $\pm0.3$, $\pm0.2$, $\pm0.1$, *etc.,* and includes all of the values that are equivalent or similar to those following the values, but the range is not limited thereto.

The interval between administrations may be 1 week, 2 weeks, 4 weeks, 6 weeks, 8 weeks, 10 weeks, 12 weeks, 16 weeks, 20 weeks, 24 weeks, 28 weeks, 36 weeks, or more, and each administration interval may be the same or different. For example, the interval between the second and third administrations may be longer than the interval between the first and second administrations, but is not limited thereto.

The period during which the pharmaceutical composition of the present invention is administered may be 6 weeks or more, 8 weeks or more, 12 weeks or more, 20 weeks or more, 24 weeks or more, 28 weeks or more, 36 weeks or more, or 48 weeks or more, *etc.,* but the composition may be administered without limitation of period as long as multiple sclerosis is improved.

The pharmaceutical composition according to the present invention may include ocrelizumab in an amount of about 1 mg/mL or more, about 5 mg/mL or more, about 10 mg/mL or more, about 5 mg/mL or more, about 15 mg/mL or more, about 20 mg/mL or more, about 30 mg/mL or more, about 40 mg/mL or more, about 50 mg/mL or more, about 60 mg/mL or more, 70 mg/mL or more, about 80 mg/mL or more, about 90 mg/mL or more, about 100 mg/mL or more, about 110 mg/mL or more, about 120 mg/mL or more, but the amount is not limited thereto. Specifically, the pharmaceutical composition of the present invention may include ocrelizumab in an amount of 10 mg/mL to 350 mg/mL, 30 mg/mL to 350 mg/mL, or 60 mg/mL to 350 mg/mL, but the amount is not limited thereto.

The pharmaceutical composition of the present invention may be repeatedly administered to patients with multiple sclerosis at intervals of 2 weeks to 36 weeks, but the interval is not limited thereto. In addition, the pharmaceutical composition may be administered once, twice or more multiple times.

Specifically, the pharmaceutical composition of the present invention may be administered once in every 2 to 36 weeks, every 2 to 28 weeks, every 2 to 24 weeks, every 2 to 12 weeks, every 2 to 6 weeks, every 2 to 4 weeks, every 4 to 28 weeks, every 4 to 24 weeks, every 4 to 12 weeks, every 4 to 8 weeks, every 8 to 36 weeks, every 8 to 28 weeks, every 8 to 24 weeks, every 8 to 20 weeks, every 8 to 16 weeks, every 12 to 36 weeks, every 12 to 28 weeks, every 12 to 24 weeks, or every 12 to 20 weeks, but the interval is not limited thereto. Additionally, the total volume of the formulation may be administered within 1 to 10 minutes, 2 to 6 minutes, or 2 to 4 minutes at the time of a single administration. More specifically, the composition may be administered at 2 mL/min, *i.e.,* for example, about 240 mg/min, and the administration frequency is not limited.

The pharmaceutical composition of the present invention may be administered at doses and intervals as follows, but the doses and intervals are not limited thereto:

In the composition, ocrelizumab is subcutaneously administered at a dose of about 40 mg to 1,200 mg about every 2 to 36 weeks.

More specifically, in the composition, ocrelizumab is subcutaneously administered at a dose of about 125 mg to 750 mg about every 4 to 28 weeks.

More specifically, in the composition, ocrelizumab is subcutaneously administered at a dose of about 375 mg to 750 mg about every 8 to 28 weeks.

Most specifically, in the composition, ocrelizumab is subcutaneously administered at a dose of about 250 mg to 500 mg about every 4 to 20 weeks.

In still another specific embodiment, the pharmaceutical composition of the present invention may be administered at doses and intervals as follows, but the doses and intervals are not limited thereto:

In the composition, ocrelizumab is subcutaneously administered at a dose of about 40 mg to 1,200 mg about every 2 to 36 weeks.

Specifically, in the composition, ocrelizumab is subcutaneously administered at a dose of about 60 mg about every 2 weeks.

More specifically, in the composition, ocrelizumab is subcutaneously administered at a dose of about 125 mg about every 4 weeks.

More specifically, in the composition, ocrelizumab is subcutaneously administered at a dose of about 250 mg about every 8 weeks.

More specifically, in the composition, ocrelizumab is subcutaneously administered at a dose of about 375 mg about every 12 weeks.

More specifically, in the composition, ocrelizumab is subcutaneously administered at a dose of about 500 mg about every 16 weeks.

More specifically, ocrelizumab in the composition is subcutaneously administered at a dose of about 600 mg about every 20 weeks.

More specifically, in the composition, ocrelizumab is subcutaneously administered at a dose of about 750 mg about every 24 weeks.

More specifically, ocrelizumab in the composition is subcutaneously administered at a dose of about 1,125 mg about every 36 weeks.

In yet another specific embodiment, the pharmaceutical composition of the present invention may be administered at doses and intervals as follows, but the doses and intervals are not limited thereto:

Specifically, in the composition, ocrelizumab is subcutaneously administered at a dose of about 125 mg every 4 weeks, except that the initial administration is carried out in a way that about 62.5 mg of ocrelizumab is administered twice at 2-week intervals.

More specifically, in the composition, ocrelizumab is subcutaneously administered at a dose of about 250 mg every 8 weeks, except that the initial administration is carried out in a way that about 125 mg of ocrelizumab is administered twice at 2-week intervals.

More specifically, in the composition, ocrelizumab is subcutaneously administered at a dose of about 375 mg every 12 weeks, except that the initial administration is carried out in a way that about 187.5 mg of ocrelizumab is administered twice at 2-week intervals.

More specifically, in the composition, ocrelizumab is subcutaneously administered at a dose of about 500 mg every 16 weeks, except that the initial administration is carried out in a way that about 250 mg of ocrelizumab is administered twice at 2-week intervals.

More specifically, in the composition, ocrelizumab is subcutaneously administered at a dose of about 600 mg every 20 weeks, except that the initial administration is carried out in a way that about 300 mg of ocrelizumab is administered twice at 2-week intervals.

More specifically, in the composition, ocrelizumab is subcutaneously administered at a dose of about 750 mg every 24 weeks, except that the initial administration is carried out in a way that about 375 mg of ocrelizumab is administered twice at 2-week intervals.

More specifically, in the composition, ocrelizumab is subcutaneously administered at a dose of about 1,125 mg every 36 weeks, except that the initial administration is carried out in a way that about 490 mg of ocrelizumab is administered twice at 2-week intervals.

According to a clinical study of ocrelizumab for patients with multiple sclerosis and rheumatoid arthritis (Study # ACT2847g), when $2\times10$ mg, $2\times50$ mg, $2\times200$ mg, $2\times500$ mg, and $2\times1,000$ mg of ocrelizumab was injected intravenously into the human body, immediate blood B cell depletion was observed ($2\times$ means double injection on days 1 and 15). The duration of efficacy maintenance until blood B cell regeneration (repletion) increased as the dose increased, but there was no significant difference in the duration at doses of $2\times200$ mg or higher. According to another clinical study (Study # WA21092, WA21093, WA25046) in patients with multiple sclerosis, the IRR rates according to the intravenous infusion between single infusion of 600 mg and double infusions of 300 mg (double infusions, Day 1 and Day 15) were similar, the regimen of single infusion was approved from the second injection except for the initial injection. In the present invention, dosage regimens of ocrelizumab using the subcutaneous injection route were established in consideration of the effects of the dosage regimens of human intravenous injection of ocrelizumab on the efficacy and safety, and the efficacy and safety of ocrelizumab were predicted based on the pharmacokinetics exhibited when the composition of the present invention was applied via subcutaneous administration to animals.

Specifically, in the Examples of the present invention, after setting the formula for the bioavailability of subcutaneous injection from the results obtained by comparing the pharmacokinetics of intravenous and subcutaneous injections, it was applied to parameters related to drug exposure when predicting dosage regimens for each human subcutaneous injection scenario. In order to apply the composition of the present invention to animal experiments, the range of single intravenous experimental doses (0.5 mg/kg, 5 mg/kg, 50 mg/kg) in rats reported in nonclinical pharmacokinetic data of ocrelizumab (FDA nonclinical pharmacology reviews, application number: 761053 Orig1s000) was considered. In addition, absorption kinetics and clearance kinetics were observed and compared when the ocrelizumab composition of the present invention was administered intravenously or subcutaneously at concentrations of 1 mg/kg, 4 mg/kg, 6 mg/kg, 8 mg/kg, 10 mg/kg, and 20 mg/kg so as to include the range of intravenous doses approved for human body. As a result, as shown in Examples 1 to 6, it was confirmed that the pharmacokinetic parameters of intravenous infusion obtained from non-clinical studies reported upon approval of ocrelizumab were consistent, and there was no significant difference in the pharmacokinetics of clearance phase between intravenous and subcutaneous administration in rats. In addition, parameters necessary for predicting human pharmacokinetics in the absorption phase during subcutaneous injection were derived from animal experiments.

The pharmaceutical composition according to the present invention may be subcutaneously administered, including ocrelizumab and hyaluronidase, and may further include a buffer, a surfactant, a stabilizer, and a combination thereof for subcutaneous administration. However, additional components may be included in the present invention without limitation as long as they can be subcutaneously administered while exerting pharmacological effects.

Specifically, the buffer is not limited as long as it is a component that maintains the pH of the composition within the range of pH 3.5 to pH 7.5, and non-limiting examples may include one or more selected from the group consisting of histidine, Tris, Bis-Tris, phosphate, acetic acid, succinic acid, *etc.* The buffer may have a pH of 3.5 to 7.5, a pH of 4 to 7, a pH of 4 to 6, a pH of 4.5 to 6.5, a pH of 5 to 7, or a pH of 5.5 to 7.5.

Specifically, the surfactant refers to a substance which reduces the surface tension of a fluid in which it is dissolved, and the surfactant may be a non-ionic surfactant. Specifically, the non-ionic surfactant may include one or more selected from the group consisting of polysorbates such as polysorbate 20, polysorbate 28, polysorbate 40, polysorbate 60, polysorbate 65, polysorbate 80, polysorbate 81, polysorbate 85, *etc.;* poloxamers including, for example, poloxamer 188, also known as poloxalkol or poly(ethylene oxide)-poly(propylene oxide), poloxamer 407 or polyethylene-polypropylene glycol, *etc.;* and polyethylene glycol (PEG).

More specifically, the nonionic surfactant may include polysorbate 20, polysorbate 80, or poloxamer in an amount of 0% (w/v) to 0.2% (w/v).

The stabilizer refers to a substance that stabilizes the native conformation of an antibody and/or prevents or reduces the physical or chemical degradation of the antibody, but is not limited thereto. Non-limiting examples may include any one or more selected from the group consisting of polyols (e.g., sorbitol, glycerol, mannitol, xylitol, maltitol, lactitol, erythritol and threitol), sugars (e.g., fructose, glucose, glyceraldehyde, lactose, arabinose, mannose, xylose, ribose, rhamnose, galactose maltose, sucrose, trehalose, sorbose, sucralose, melezitose and raffinose), sodium chloride, and amino acids (e.g., glycine, methionine, proline, lysine, arginine, histidine, or glutamic acid). More specifically, the stabilizer may include any one or more selected from the group consisting of sucrose, trehalose, mannitol, and glucose.

As one specific example of the pharmaceutical composition of the present invention, a formulation having the composition of chloroacetic acid buffer (20 mM sodium acetate), one or two or more stabilizers (e.g., 106 mM trehalose, and optionally 85 mM sodium chloride for secondary stabilizer), and a nonionic surfactant (PX188, 0.2%), with a pH adjusted to 5.3 may be included, but is not limited thereto.

As another specific example of the pharmaceutical composition of the present invention, a stable drug formulation of ocrelizumab may include a formulation including about 30 mg/mL to 350 mg/mL of ocrelizumab, for example, about 30 mg/mL to 100 mg/mL of ocrelizumab (e.g., Humanized 2H7.v16) (as specific examples, about 30 mg/mL, about 50 mg/mL or about 100 mg/mL); about 1 mM to 100 mM of a buffer (e.g., sodium acetate) at pH 5.5±2.0 (e.g., pH 5.3); about 15 mM to 250 mM of a stabilizer or a mixture of two or more stabilizers (including trehalose, e.g., about 8% trehalose dihydrate); about 0.01% (w/v) to 0.1% (w/v) of a nonionic surfactant; and at least one molecule of hyaluronidase (as specific examples, SEQ ID NO: 1 or SEQ ID NO: 2) in an amount of about 1,000 U/mL to 12,000 U/mL, but is not limited thereto.

Unless specified otherwise in the present invention, the description in the detailed description or claims with respect to ocrelizumab according to the present invention may be applied to the scope including all of the forms, which include not only the corresponding ocrelizumab, but also the salts of the corresponding ocrelizumab (e.g., pharmaceutically acceptable salts thereof), or solvates thereof. Accordingly, even in a case where ocrelizumab is described in the present invention, the description may also be equally applied to a particular salt thereof, a particular solvate thereof, and a particular solvate of the particular salt thereof. These salts may be, for example, in a form where any pharmaceutically acceptable salts are used. The kind of the salt is not particularly limited. However, the salt is preferably one that is safe and effective to a subject, e.g., a mammal, but is not particularly limited thereto.

The term "pharmaceutically acceptable" refers to a material which can be effectively used for the intended use within the scope of pharmaco-medical decision without inducing excessive toxicity, irritation, allergic responses, etc.

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, etc. Examples of the salts derived from suitable bases may include alkali metals such as sodium, potassium, etc.; alkali earth metals such as magnesium; and ammonium, etc.

Additionally, as used herein, the term "solvate" refers to a complex formed between the ocrelizumab according to the present invention or a salt thereof, and a solvent molecule.

The pharmaceutical composition including ocrelizumab of the present invention is a pharmaceutical composition for preventing or treating multiple sclerosis, and may be subcutaneously administered.

Multiple sclerosis (MS) of the present invention is a chronic disease that occurs in the central nervous system consisting of the brain, spinal cord, and optic nerve, and is an autoimmune disease in which the patient's immune system attacks healthy cells and tissues. The most common symptoms include sensory symptoms and movement disorders. It is known that the sensory symptoms appear in the form of abnormal sensations such as numbness, tingling, burning, etc., and the movement disorders appear in various ways, such as hemiplegia, paraplegia, or quadriplegia, etc. In the development of therapeutics for multiple sclerosis, CD20 has been studied as an important treatment target, and in this respect, therapeutics using the anti-CD20 antibody, typically ocrelizumab, which is a drug that depletes CD20+ B cells, have been studied.

As used herein, the term "prevention" refers to all activities that inhibit or delay the occurrence of multiple sclerosis in a subject by administering ocrelizumab or the composition including the same.

As used herein, the term "treatment" refers to all activities that improve or advantageously change the symptoms of multiple sclerosis in a subject by administering ocrelizumab or the composition including the same.

As used herein, the term "subject" refers to a subject having multiple sclerosis or suspected of having multiple sclerosis, and may mean mammals including humans, rats, livestock, etc., but any subject which can be treated with ocrelizumab or the composition including the same is included without limitation.

The total effective dose of the composition of the present invention may be administered to a patient in a single dose or may be administered for a long period of time in multiple doses according to a fractionated treatment protocol. In the pharmaceutical composition of the present invention, the content of active ingredient(s) may vary depending on the severity of the disease. The effective dose of ocrelizumab is determined considering various factors including patient's age, body weight, health conditions, sex, disease severity, diet, and excretion rate, as well as administration route and treatment frequency of the pharmaceutical composition. In this respect, those skilled in the art may easily determine the effective dose suitable for a particular use of the composition of the present invention. The pharmaceutical composition according to the present invention is not particularly limited to the formulation type, administration route and mode, as long as it shows the effects of the present invention.

Formulations of the pharmaceutical composition of the present invention may be variously prepared by mixing with pharmaceutically acceptable excipients. In a specific embodiment, the pharmaceutical composition of the present invention may be prepared into a formulation suitable for administration of ocrelizumab, in particular, subcutaneous administration. In addition, the pharmaceutical composition of the present invention may be prepared into a stabilized formulation capable of maintaining the pharmacological activity of ocrelizumab, but is not limited thereto.

The pharmaceutically acceptable excipient may include, for injection preparations, a buffering agent, a preservative, an analgesic, a solubilizing agent, an isotonic agent, a stabilizing agent, *etc.,* which may be combined to be used; and for topical administrations, a base, an excipient, a lubricant, a preservative, *etc.,* although it is not particularly limited thereto.

The pharmaceutically acceptable excipient may be included in a sufficient amount to exhibit a therapeutic effect and in an amount and type that do not cause adverse effects, and may be easily determined by a skilled person in the art based on the factors well known in the medical field, such as the kind of disease, age, body weight, health status, sex, drug sensitivity of a patient, administration route, administration method, administration frequency, duration of treatment, a drug to be mixed or administered simultaneously in combination, *etc.*

Meanwhile, examples of suitable carriers, excipients, and diluents for formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anti-coagulant, a lubricant, a humectant, a fragrance, a preservative, *etc.*

Additionally, the ocrelizumab and hyaluronidase may be used by being mixed with various pharmaceutically acceptable carriers such as physiological saline or organic solvents. To increase stability or absorptivity, carbohydrates, such as glucose, sucrose, or dextrans, antioxidants, such as ascorbic acid or glutathione, chelating agents, low-molecular weight proteins, or other stabilizers, *etc.* may be used as pharmaceutical drugs.

For example, the pharmaceutical composition of the present invention may be formulated into unit-dose ampoules or multi-dose forms. The pharmaceutical composition may also be formulated into solutions, suspensions, tablets, pills, capsules, sustained-release formulations, *etc.*

Specifically, the pharmaceutical composition may be formulated into an injectable preparation for subcutaneous administration.

The injectable preparation is directly administered into the human body via a subcutaneous route, a blood vessel (intravascular injection), or a muscle (intramuscular injection) without undergoing absorption in the gastrointestinal tract or metabolism in the liver, and thus effects thereof may be directly obtained regardless of solubility or bioavailability thereof.

As used herein, the term "administration" refers to the introduction of ocrelizumab into a patient by any appropriate method, and the administration route is not particularly limited, but may be any conventional route that enables delivery of ocrelizumab to the target in the body, and may be administered, for example, by intraperitoneal administration, intravenous administration, intramuscular administration, subcutaneous administration, intradermal administration, oral administration, topical administration, intranasal administration, intrapulmonary administration, or intrarectal administration, *etc.*

In the present invention, the "administration" may mean subcutaneous administration.

Additionally, the pharmaceutical composition of the present invention may be formulated in a unit dosage form suitable for administration to the body of a patient, specifically in a form useful for administration of protein medicines, according to a method commonly used in the pharmaceutical field to be subcutaneously administered.

As used herein, intravenous administration is an administration mode in which a drug is directly injected into a vein, and is widely used because it can rapidly induce drug effects throughout the body as an accurate dose of the drug is rapidly administered. However, intravenous injection has problems of side effects caused by injection solutions and side effects caused by drugs directly injected into the bloodstream.

As used herein, subcutaneous administration refers to an administration of a pharmaceutical composition beneath the skin, and may mean an administration mode in which a drug is injected by inserting a needle into adipose tissue just beneath the skin. The injected drug is delivered into the body through capillaries.

The pharmaceutical composition including ocrelizumab of the present invention can solve problems caused by intravenous administration with subcutaneous administration.

The above description may be applied to other embodiments or aspects of the present invention, but is not limited thereto.

Another aspect for implementing the present invention provides a pharmaceutical composition for preventing or treating multiple sclerosis, including 40 mg to 1,200 mg of ocrelizumab; and hyaluronidase. The pharmaceutical composition may be a pharmaceutical composition for one-time administration, and may be administered every 2 to 24 weeks, 2 to 12 weeks, and 2 to 8 weeks, but is not limited thereto.

Additionally, the pharmaceutical composition may be a pharmaceutical composition for subcutaneous administration, but is not limited thereto.

The 40 mg to 1,200 mg of ocrelizumab in the pharmaceutical composition may be administered in a single dose or divided doses suitable for subcutaneous administration, but is not limited thereto.

Specific examples of the pharmaceutical composition may include a pharmaceutical composition including about 10 mg/mL to 350 mg/mL of ocrelizumab; about 1 mM to 100 mM of a buffer at pH 5.5±2.0; about 1 mM to 500 mM of a stabilizer or a mixture of two or more stabilizers; and 0.01% to 0.1% of a nonionic surfactant; and may optionally include 5 mM to 25 mM of methionine, but are not limited thereto.

The pharmaceutical composition according to the present invention may include ocrelizumab in an amount of about 1 mg/mL or more, about 5 mg/mL or more, about 10 mg/mL or more, about 5 mg/mL or more, about 15 mg/mL or more, about 20 mg/mL or more, about 30 mg/mL or more, about 40 mg/mL or more, about 50 mg/mL or more, about 60 mg/mL or more, 70 mg/mL or more, about 80 mg/mL or more, about 90 mg/mL or more, about 100 mg/mL or more, about 110 mg/mL or more, or about 120 mg/mL or more, but the amount is not limited thereto.

In addition, the pharmaceutical composition may include hyaluronidase in an amount of about 50 U/mL to about 12,000 U/mL, or about 1,000 U/mL to about 5,000 U/mL, but the amount is not limited thereto.

Still another aspect for implementing the present invention provides a pharmaceutical composition for CD20 targeted therapy, including ocrelizumab; and hyaluronidase.

The pharmaceutical composition is characterized in that, in the composition, ocrelizumab is subcutaneously administered at a dose of 40 mg to 1,200 mg to patients targeted for CD20 targeted therapy.

The ocrelizumab, hyaluronidase, treatment, administration, *etc.* are as described above.

As used herein, the "CD20 targeted therapy" may refer to all activities that improve or advantageously change the symptoms of a related disease by targeting CD20 antigen-expressed B cells and interfering with their formation. In particular, the diseases that can be treated by targeting CD20 include, in addition to multiple sclerosis, rheumatoid arthritis (RA), non-Hodgkin's lymphoma (NHL), chronic lymphocytic leukemia (CLL), or B-cell acute lymphoblastic leukemia (B-cell acute leukemia), *etc.*

Yet another aspect for implementing the present invention provides a formulation for subcutaneous administration for the prevention or treatment of multiple sclerosis including 40 mg to 1,200 mg of ocrelizumab; and hyaluronidase.

The ocrelizumab, hyaluronidase, multiple sclerosis, prevention, treatment, administration, *etc.* are as described above.

The formulation for subcutaneous administration for the prevention or treatment of multiple sclerosis of the present invention may refer to a pharmaceutical composition formulated such that 40 mg to 1,200 mg of ocrelizumab; and hyaluronidase can be subcutaneously administered, but is not limited thereto. Specifically, the formulation may be an injection preparation, but is not limited thereto.

For example, with respect to the formulation of the present invention, 40 mg to 1,200 mg of ocrelizumab may be subcutaneously administered once, and the interval between administrations may be 2 to 24 weeks, but is not limited thereto.

Even another aspect for implementing the present invention provides a method for preventing or treating multiple sclerosis, including: subcutaneously administering 40 mg to 1,200 mg of ocrelizumab or a pharmaceutical composition including the same to a subject. The pharmaceutical composition may further include hyaluronidase, but is not limited thereto.

The above method is a method for obtaining excellent preventive or therapeutic effects of multiple sclerosis while subcutaneously administering 40 mg to 1,200 mg of ocrelizumab to a subject.

In addition, the method may further include administering hyaluronidase, but is not limited thereto. The hyaluronidase may be administered simultaneously, sequentially, or in reverse order with ocrelizumab, but is not limited thereto.

The ocrelizumab, hyaluronidase, pharmaceutical composition, formulation, administration, multiple sclerosis, prevention, treatment, *etc.* are as described above.

For example, the method of the present invention may be a method of administering the above-described pharmaceutical composition of the present invention to a subject, but is not limited thereto.

Hereinafter, the present invention will be described in more detail by way of Examples. However, these Examples are for illustrative purposes only and the scope of the invention is not limited by these Examples.

### Example 1. Preparation of Subcutaneous Formulations Including Ocrelizumab and Hyaluronidase

Subcutaneous formulations including ocrelizumab and hyaluronidase were prepared.

Specifically, ocrelizumab was prepared at concentrations of 14 mg/mL, 21 mg/mL, and 28 mg/mL, such that ocrelizumab can be administered intravenously or subcutaneously at doses of 1 mg/kg, 4 mg/kg, 6 mg/kg, 8 mg/kg, 10 mg/kg and 20 mg/kg. Subcutaneous formulations including 1,000 units/mL of hyaluronidase, which contains sequences 38 to 468 of SEQ ID NO: 2 were prepared. Formulations including sodium acetate buffer, 4% trehalose dihydrate, and a nonionic surfactant as a buffer in the form of intravenous injection, and formulations including sodium acetate buffer, trehalose dihydrate, sodium chloride, and a nonionic surfactant as a buffer in the form of subcutaneous injection were further prepared.

## Example 2. Establishment of Quantification for Ocrelizumab in Laboratory Animal Serum

To perform quantitative experiments for ocrelizumab in laboratory animal serum, Anti-Human IgG (Fc specific) Ab from goat (Sigma-Aldrich, Cat. No. 71289) was first diluted to 0.5 μg/mL in 1×D-PBS (WELGENE, Cat. No. LB001-02), and then 100 μL of the antibody was added to each well in a 96-well immunoplate (Maxisorp; Thermo Fisher, Cat. No. 439454) and incubated overnight at 4°C to coat the immunoplate. Thereafter, the coated samples were removed and washed 5 times with 300 μL of 1×D-PBST (0.05% Polysorbate 20 in 1×D-PBS), and then, 300 μL of a blocking buffer (5% BSA in 1×D-PBST) was added to each well of the coated immunoplate and reacted at 25°C for 2 hours to perform blocking.

As the antibody standard solution, an ocrelizumab solution of known content was used. The antibody standard solution was prepared by adding the same amount of rat serum (blank serum) as the sample in TPBSA-1 buffer (0.05% Sodium azide, 0.1% Polysorbate 20, 1% BSA in 1×D-PBS Buffer) to be spiked. The samples were serial diluted by 1/2 in the TPBSA-1 buffer spiked with the antibody standard solution in the calibration range of 0.12 ng/mL to 124.28 ng/mL, which was confirmed by duplication (Table 1), and the samples were prepared by diluting in TPBSA-1 buffer. 100 μL of the diluted standard and samples were added to each well of an immunoplate and incubated for 1 hour at 25°C.

[Table 1]

| : *TPBSA1 spiking 1/1500 | | | | | |
|---|---|---|---|---|---|
| | serum | TPBSA1 (μL) | Total Vol. (ul) | dilution factor | total DF |
| | 5 | 495 | 500 | 100 | 100 |
| | 400 | 5,600 | 6,000 | 15 | 1,500 |
| | | | | | |
| sample | | | | | |
| G1 | serum | TPBSA1 (μl) | Total Vol. (ul) | dilution factor | total DF |
| | 5 | 495 | 500 | 100 | 100 |
| | 40 | 560 | 600 | 15 | 1,500 |

After the incubation reaction of the sample was completed, the samples were removed and washed 5 times with 300 μL of 1×D-PBST, and then 100 μL of Peroxidase AffiniPure F(ab')2 Fragment Goat Anti-Human IgG (Jackson, Cat. No. 109-036-006), which was diluted at 1:100,000 in TPBSA-2 buffer (0.05% Polysorbate 20, 1% BSA, 0.05% Proclin® 300 in 1×D-PBS), was loaded onto each well of an immunoplate and incubated at 25°C for 1 hour for detection. After incubation, the samples were removed from the immunoplate and washed 5 times with 300 μL of 1×D-PBST, and then 100 μL of TMB solution (Solution A: Solution B = 1:1 (V/V); Seracare, Cat. No. 5120-0048, 5120-0037) was loaded onto an immunoplate increments and reacted in the dark at 25°C for 30 minutes. After completion of the TMB reaction, 100 μL of 1 M $H_2SO_4$ was loaded onto a 96-well immunoplate without removing the TMB solution to terminate the reaction. After confirming that the TMB reaction was stopped and yellow color appeared, the absorbance at 450 nm was measured using a microplate reader.

## Example 3. Pharmacokinetic Analysis and Comparison of Bioavailability of Intravenous and Subcutaneous Injection of Ocrelizumab

The pharmacokinetic properties of the antibodies were compared for the cases when the formulation for subcutaneous administration including the ocrelizumab of the present invention prepared in Example 1 and hyaluronidase was administered to a 9-week-old Sprague-Dawley (SD) rat model and when ocrelizumab was intravenously administered and in plasma.

A total of 20 male, 8-week-old SD rats were obtained from ORIENT BIO Inc. (322 Galmachi-ro, Jungwon-gu, Seongnam-si, Gyeonggi-do) and subjected to a 4-day acclimatization period. The rats were divided into groups on the day before administration for all animals. Four groups of four males were selected as pharmacokinetics experiment groups, which were close to the average body weight, and the groups were divided so that the body weight was equal. For tail vein and intradermal administration, the test substance prepared in Example 1 was injected using a 26G, 1 mL syringe. All animals were fasted for about 16 hours or more before administration, while water was given freely. Feed was given 4 hours after administration. In SD rats, the drug was injected intradermally into the upper part of the skin to mimic the subcutaneous injection route including hyaluronidase, and in the case of intravenous injection, the drug was injected through the tail vein. The pharmacokinetic experiment was carried out as follows: the test material prepared in Example 1 was injected into male rats in a total of 4 groups, *i.e.,* 1 group of 4 rats for intravenous administration, and 3 groups of 4 rats for intradermal administration via intravenous administration at 4 mg/kg (G1) and subcutaneous administration at 4 mg/kg (G2), subcutaneous administration at 6 mg/kg (G3), and subcutaneous administration at 8 mg/kg (G4) (Table 2).

9-week-old SD rats were injected in divided doses of 90 $\mu$L to 100 $\mu$L into the left and right sides of the abdomen, and about 500 $\mu$L of blood was collected using a 1 mL syringe from the jugular vein at 0 hour before administration, 0.072, 0.5, 1, 4, and 8 hours, and 1, 2, 3, 7, 14, 21, and 28 days after intravenous and intradermal administration in all experimental groups, and centrifuged at 3,500 rpm for 10 minutes, and then serum was isolated and collected. The isolated serum was dispensed into three tubes in volumes of 100 $\mu$L, 100 $\mu$L, and 50 $\mu$L, and stored frozen (-70°C) until analysis. Blood samples were quantified and analyzed by the ELISA method established in Example 2.

Table 2 shows the main characteristics of the pharmacokinetic analysis, and the blood concentration of ocrelizumab versus time is shown in FIG. 1.

[Table 2]

| Parameter | IV_4mpk (G1) | SC_4 mpk (G2) | SC_6mpk (G3) | SC_8 mpk (G4) |
|---|---|---|---|---|
| $t_{1/2}$ (day) | 10.88 $\pm$ 1.94 | 6,92 $\pm$ 1.53 | 9.39 $\pm$ 1,67 | 10.35 $\pm$ 2,97 |
| $C_0$ ($\mu$g/mL) | 88.54 $\pm$ 20.33 | - | - | |
| $C_{max}$ (day) | - | 20.72 +1.19 | 34.15 $\pm$ 2.85 | 50.97 $\pm$ 3.79 |
| $T_{max}$ (day) | - | 2.75 $\pm$ 0.5 | 2.25 $\pm$ 0.96 | 1.5 $\pm$ 1 |
| $AUC_{last}$ ($\mu$g.day/mL) | 309.28 $\pm$ 50.74 | 200.98 $\pm$ 6.01 | 452.18 $\pm$ 59.25 | 540.46 $\pm$ 220.7 |
| $AUC_{inf}$ ($\mu$g.day/mL) | 388.54 $\pm$ 31.59 | 271.13 $\pm$ 30.21 | 570.33 $\pm$ 61.1 | 830.1 $\pm$ 157.67 |
| $V_2$ or $V_2/F$ (mL/kg) | 162.68 $\pm$ 33.56 | 146.02 $\pm$ 19.03 | 142.32 $\pm$ 18.35 | 142.01 $\pm$ 22.56 |
| CL or CL/F (mL/day/kg) | 10.34 + 0.81 | 14.89 $\pm$ 1.68 | 10.61 $\pm$ 1.11 | 9.96 $\pm$ 2.29 |
| $V_{SS}$ (mL/kg) | 146.77 $\pm$ 23.11 | - | - | - |
| Absolute BA (%) | - | 69.78 $\pm$ 7.77 | 97.86 $\pm$ 10.48 | 106.82 $\pm$ 20.29 |

As a result, it was confirmed that the experimental groups (G2 to G4) injected subcutaneously showed equivalent half-lives in the clearance phase up to the drug concentration of 8 mg/kg compared to the control group (G1) injected intravenously with ocrelizumab. In the case of subcutaneous injection, the absolute bioavailability was found to be 70% to 100% compared to that of intravenous injection. In addition, the absolute bioavailability evaluated using the area under the curve (AUC) from 0 to 14 days was calculated to be 82% to 101% in the range of ocrelizumab concentrations tested. Therefore, it was confirmed that most of ocrelizumab was absorbed into the body in the formulations of subcutaneous injection including hyaluronidase.

The result of the pharmacokinetic experiment means that when the pharmaceutical composition according to the present invention is administered subcutaneously, it shows efficacy equivalent to that of intravenous administration, further suggesting that subcutaneously administered ocrelizumab may exhibit a therapeutic effect on multiple sclerosis.

### Example 4. Pharmacokinetic Analysis and Comparison of Bioavailability of Intravenous and Subcutaneous Injection of Ocrelizumab in Extended Doses

In the pharmacokinetic analysis experiment in the extended doses of Example 4 using SD rats, animal breeding, administration mode, blood collection method, and analysis method were performed in the same manner as in Example 3.

Specifically, the pharmacokinetics of a total of 8 groups were analyzed by injecting the test material into male SD rats in 4 groups of 4 rats for intravenous administration via intravenous administration at 1 mg/kg (G1), intravenous administration at 4 mg/kg (G2), intravenous administration at 10 mg/kg (G3), and intravenous administration at 20 mg/kg (G4), and into male SD rats in 4 groups of 4 rats for subcutaneous administration via subcutaneous administration at 1 mg/kg (G5), subcutaneous administration at 4 mg/kg (G6), subcutaneous administration at 10 mg/kg (G7), and subcutaneous administration at 20 mg/kg (G8), and the results are shown in FIG. 2

As a result of analyzing the pharmacokinetics of the intravenous administration groups G1 to G4, the body clearance rate (CL, mL/day/kg) decreased as the dose increased. As a result, the total blood exposure per dose (AUC/dose) increased in the 10 mg/kg (G3) and 20 mg/kg (G4) groups compared to the 1 mg/kg (G1) and 4 mg/kg (G2) groups (Table 3, FIG. 2).

As a result of analyzing the pharmacokinetics of the subcutaneous administration groups G5 to G8, in the case of subcutaneous administration, the effect on the dose was relatively less than that of intravenous administration, but the absorption rate was slightly delayed in the 20 mg/kg group. Upon subcutaneous administration, the overall absorption kinetics was constant regardless of the dose (Table 4, FIG. 3). In addition, the half-life was the same when comparing the clearance phase of the intravenous administration versus subcutaneous administration at the same dose, suggesting that changing from intravenous administration to the subcutaneous injection of the present invention showed similar pharmacokinetics. Therefore, the results mean that the dosage regimen, such as a change in administration interval, *etc.,* may be set similarly (FIG. 4).

Under the assumption that the bioavailability (AUC/dose) for intravenous injection is 100%, the relative absolute bioavailability for subcutaneous injection was calculated to be about 94% at 1 mg/kg, about 120% at 4 mg/kg, and 68% and 78% at 10 mg/kg and 20 mg/kg, respectively. With the combination of Example 3 and Example 4, in the case of subcutaneous injection, absolute bioavailability was calculated to be about 100% at 1 mg/kg to 8 mg/kg and 70% to 80% at 10 mg/kg to 20 mg/kg (Table 4).

[Table 3]

| Parameter | IV_1 mg/kg (G1) | IV_4 mg/kg (G2) | IV_10 mg/kg (G3) | IV_20 mg/kg (G4) |
|---|---|---|---|---|
| $t_{1/2}$ (day) | $10.23 \pm 2.45$ | $14.1 \pm 2.06$ | $15.25 \pm 7.46$ | $7.08 \pm 2.46$ |
| $C_0$ (μg/mL) | $23.71 \pm 3.29$ | $60.38 \pm 19.42$ | $393.66 \pm 53.56$ | $615.43 \pm 52.75$ |
| $AUC_{last}$ (μg.day/mL) | $79.47 \pm 3.83$ | $299.44 \pm 25.37$ | $1256.79 \pm 132.2$ | $2260.79 \pm 532.66$ |
| $AUC_{inf}$ (μg.day/mL) | $92.94 \pm 8.67$ | $394.12 \pm 30.03$ | $1849.38 \pm 270.33$ | $2485.63 \pm 721.34$ |
| $V_2$ (L/kg) | $0.16 \pm 0.03$ | $0.21 \pm 0.03$ | $0,13 \pm 0.04$ | $0.08 \pm 0.01$ |
| CL (mL/day/kg) | $10.83 \pm 0.93$ | $10.2 \pm 0.81$ | $6.19 \pm 1.04$ | $8.46 + 2.12$ |
| $V_{SS}$ (L/kg) | $0.15 \pm 0.02$ | $0.19 \pm 0.02$ | $0.11 \pm 0.04$ | $0.09 \pm 0.01$ |
| AUClast/Dose | $79,47 \pm 3.83$ | $74.86 \pm 6.34$ | $125.68 \pm 13.22$ | $113.04 \pm 26.63$ |
| AUCinf/Dose | $92.94 \pm 8.67$ | $98.53 \pm 7.51$ | $164.94 \pm 27.03$ | $124.28 \pm 36.07$ |

[Table 4]

| Parameter | SC_1 mg/kg (G5) | SC_4 mg/kg (G6) | SC_14 mg/kg (G7) | SC_20 mg/kg (G8) |
|---|---|---|---|---|
| $t_{1/2}$ (day) | $12.83 \pm 0.15$ | $8.59 \pm 5.72$ | $8.37 \pm 4.86$ | $9.91 \pm 4.69$ |
| $T_{max}$ (day) | $2.5 \pm 0.58$ | $2.25 \pm 0.5$ | $2.25 \pm 0.5$ | $3 \pm 2.83$ |
| $C_{max}$ (μg/mL) | $4.24 \pm 0.56$ | $23.4 \pm 2.22$ | $58.93 \pm 1.51$ | $100.67 \pm 10.1$ |
| $AUC_{last}$ (μg.day/mL) | $74.54 \pm 9.77$ | $360.2 \pm 37.77$ | $944.88 \pm 72.24$ | $1577.31 \pm 281.16$ |
| $AUC_{inf}$ (μg.day/mL) | $97.34 \pm 13.63$ | $438.68 \pm 103.07$ | $1123.22 \pm 196.58$ | $1940.05 \pm 517.79$ |
| $V_2/F$ (mL/kg) | $0.19 \pm 0.02$ | $0.1 \pm 0.05$ | $0.1 \pm 0.05$ | $0.15 \pm 0.04$ |
| CL/F (mL/day/kg) | $10.44 \pm 1.57$ | $9.53 \pm 2.37$ | $9.11 \pm 1.6$ | $11.09 \pm 3.9$ |
| Cmax/Dose | $4.24 \pm 0.56$ | $5.85 \pm 0.56$ | $5.89 \pm 0.15$ | $5.03 \pm 0.51$ |
| AUClast/Dose | $74.54 \pm 9.77$ | $90.05 \pm 9,44$ | $94.49 \pm 7.22$ | $78.87 \pm 14.06$ |
| AUCinf/Dose | $97.34 \pm 13.63$ | $109.67 \pm 25.77$ | $112.32 \pm 19.66$ | $91 \pm 25.89$ |
| Absolute BA (%) | $93.8 \pm 12.29$ | $120.29 \pm 12.61$ | $68.1 \pm 11.92$ | $78.05 \pm 20.83$ |

## Example 5. Comparison of Pharmacokinetics and Bioavailability According to the Presence or Absence of Hyaluronidase in Subcutaneous Injection of Ocrelizumab

In the pharmacokinetic analysis experiment according to the presence or absence of hyaluronidase of Example 5 using SD rats, animal breeding, administration mode, blood collection method, and analysis method were performed in the same manner as in Example 3.

Specifically, a 20 mg/kg of ocrelizumab sample including hyaluronidase was prepared as a subcutaneous formulation including 1,000 U/mL of hyaluronidase, which contains sequences 38 to 468 of SEQ ID NO: 2, and sodium acetate buffer, 4% trehalose dihydrate, and a nonionic surfactant were included as buffers. A 20 mg/kg of ocrelizumab sample without hyaluronidase was prepared in the same manner as described above except that hyaluronidase was excluded.

As a result of comparing the pharmacokinetics including absorption phase kinetics and clearance phase kinetics between cases with and without hyaluronidase when ocrelizumab was administered subcutaneously at a dose of 20 mg/kg, absorption rate ($T_{max}$) was increased by hyaluronidase. In addition, it was confirmed that the bioavailability of subcutaneous injection was significantly increased by about 30% when hyaluronidase was included compared to when hyaluronidase was not included (FIG. 5).

## Example 6. Pharmacokinetic Analysis According to Repeated Administration in Intravenous Injection of Ocrelizumab and Subcutaneous Injection Including Hyaluronidase

In the pharmacokinetic analysis experiment according to the repeated administration of Example 6 using SD rats, animal breeding, administration mode, blood collection method, and analysis method were performed in the same manner as in Example 3.

Specifically, intravenous and subcutaneous injections including 20 mg/kg of ocrelizumab were injected twice at 28-day intervals into four SD rats in each group, and 600 $\mu$L of blood was collected using a 1 mL syringe at 0, 0.072, 1, 4, and 8 hours, and 1, 2, 3, 7, 10, 14, 17, 21, 24 and 28 days after the initial injection, and centrifuged at 3,500 rpm for 10 minutes to collect serum.

For the second injection, the same dose of 20 mg/kg was injected on the 28th day after the initial injection, and the time interval for collecting blood was the same as the initial injection.

Changes in the clearance phase were observed upon repeated intravenous administration by the method of the present invention, and it was confirmed that this was the result of nonlinear pharmacokinetics according to an increase in the blood concentration of ocrelizumab. During repeated administration, an increase in maximum drug concentration ($C_{max}$) and an increase in AUC were observed, indicating that drug accumulation may occur until the secondary administration (Table 5 and FIG. 6).

[Table 5]

| Parameter | IV initial administration | IV second administration |
|---|---|---|
| $t_{1/2}$ (day) | 7.08 ± 2.46 | 12.9 ± 1.34 |
| $C_0$ ($\mu$g/mL) | 615.43 ± 52.75 | 839.05 ± 92.16 |
| AUC$_{last}$ ($\mu$g.day/mL) | 2260.79 ± 532.66 | 4092.22 ± 506.87 |
| AUC$_{inf}$ ($\mu$g.day/mL) | 2485.63 ± 721.34 | 5815.3 ± 1904.98 |
| $V_Z$ (L/kg) | 0.08 ± 0.01 | 0.07 ± 0.02 |
| CL (mL/day/kg) | 8.46 ± 2.12 | 3.67 ± 0.94 |
| $V_{SS}$ (L/kg) | 0.09 ± 0.01 | 0.07 ± 0.01 |

The pharmacokinetics of the clearance phase upon repeated subcutaneous administration by the method of the present invention were similar to that of intravenous administration, and it was confirmed that the absorption rate and AUC were also similar to those of intravenous administration (Table 6 and FIG. 6).

[Table 6]

| Parameter | SC initial administration | SC second administration |
|---|---|---|
| $t_{1/2}$ (day) | 11.27 ± 4.67 | 14.58 ± 2.52 |
| $C_{max}$ ($\mu$g/mL) | 96.41 ± 6.64 | 172.72 ± 8.17 |
| $T_{max}$ (day) | 3.67 ± 3.06 | 2 ± 0 |
| AUC$_{last}$ ($\mu$g.day/mL) | 1710.05 ± 113.4 | 2683.1 ± 493.7 |
| AUC$_{inf}$ ($\mu$g.day/mL) | 2192.06 ± 145.3 | 3734.3 ± 980.56 |
| $V_Z$ or $V_Z$/F (mL/kg) | 0.15 ± 0.05 | 0.12 ± 0.01 |
| CL/F (mL/day/kg) | 9.15 ± 0.62 | 5.66 ± 1.74 |
| Absolute BA (%) | - 88.19 ± 5.85 | 150.24 ± 39.45 |

## Example 7. Prediction of Human Pharmacokinetics According to Different Dosage Regimen Scenarios for Intravenous Injection of Ocrelizumab and Subcutaneous Injection Including Hyaluronidase

When various dosage regimen scenarios were applied to the subcutaneous injection composition of the present invention, the pharmacokinetics for subcutaneous administration to the human body was mathematically predicted, and based on this, dosage regimens for subcutaneous injection, which were expected to have an effect equivalent to the human intravenous administration dose of ocrelizumab, were selected.

Specifically, the pharmacokinetics of intravenous and subcutaneous injections (composition of the present invention) were compared in animal experiments in order to predict the dosage regimens of subcutaneous injection based on clinical results of intravenous injection in humans. Analysis was conducted by dividing the obtained results into an absorption phase and a clearance phase.

In the case of the clearance phase, the pharmacokinetic parameters for each dose were similar regardless of intravenous or subcutaneous injection, and thus, the pharmacokinetics-related parameters reported in human intravenous injection clinical trials were extrapolated at a ratio of 1:1 to prediction of the pharmacokinetics of the clearance phase upon human subcutaneous injection. However, in consideration of the fact that the clearance rate (CL) shown in human intravenous injection clinical trials varied depending on the drug concentration, a Hill coefficient of 0.32 by the sigmoidal $I_{max}$ equation was applied.

In the case of the absorption phase, the bioavailability for each concentration (1 mpk, 4 mpk, 6 mpk, 8 mpk, 10 mpk, and 20 mpk) of subcutaneous injection was confirmed by the following formula, confirming that as the concentration of subcutaneous injection increased, the bioavailability decreased, compared to when the bioavailability (AUC/dose) of intravenous injection according to each injection concentration was set as 100%.

$$Y = -1.74X + 107$$

(Wherein, Y refers to the bioavailability of subcutaneous injection (%), and X refers to dose (mg/kg).)

The above bioavailability was applied to parameters (AUC and $C_{max}$) related to drug exposure when predicting the dosage regimens for each human subcutaneous injection scenario.

As a result of the pharmacokinetic analysis according to drug concentration upon intravenous and subcutaneous injections in SD rats confirmed in Examples 1 to 6, it was confirmed that there was no change in drug clearance (Clearance CL, L/day) regardless of the administration route at the same drug concentration. In addition, considering the pharmacokinetics (Study number ACT2847g, Study number WA18230, European Medicines Agency, Assessment Report for Ocrevus) reported in animal experiments confirmed in above Examples and intravenous clinical trials of ocrelizumab, it was confirmed that the clearance rate (CL, L/day) of the drug decreased and the half-life increased as the concentration of the drug increased. Therefore, in order to predict the pharmacokinetics of ocrelizumab in serum, a 2-compartment model divided into a central compartment and a peripheral compartment was applied, and the clearance rate was changed according to the sigmoidal $I_{max}$ model in order to reflect the dose dependence of the pharmacokinetics described above. Under the assumption that 400 mg, 1,000 mg, 1,500 mg, and 2,000 mg were administered to the human body based on the analysis of the clearance rate of the above-mentioned drug and the reported human pharmacokinetics of the existing intravenous injection of ocrelizumab, the parameters of the applied pharmacokinetic model are summarized in Table 7. The established model was applied to the pharmacokinetic prediction program (Berkeley Madonna) to compare the predicted blood concentration of ocrelizumab during intravenous injection with the actual value, and as a result, it was confirmed that the developed model well reflected the actual phenomenon (FIG. 7).

[Table 7]

| Parameter | Description | Unit | Value |
|---|---|---|---|
| V1 | Volume of distribution of Central compartment | L | 2.91 |
| V2 | Volume of distribution of Peripheral compartment | L | 3.62 |
| CLD | Distribution clearance | L/day | 0.34 |
| $CL_0$ | Initial clearance at concentration of 0 | L/day | 0.4 |
| $IC_{50}$ | Concentration for half maximal inhibition | mg/L | 33 |
| $I_{max}$ | Maximum fold change in CL over concentration | - | 0.43 |
| HILL | Hill coefficient of sigmoidal $I_{max}$ equation | - | 3.2 |

In the case of subcutaneous injection in the form of an antibody including hyaluronidase, it was assumed that the time to reach the maximum drug concentration ($T_{max}$) was about 48 hours, considering the correlation with the human absorption kinetics. In the case of subcutaneous injection according to the present invention, the SD rat experiment showed about 80% bioavailability compared to intravenous administration at a dose of 10 mg/kg or more overall. Based on the above absorption kinetics and the pharmacokinetic model of the clearance phase, the pharmacokinetics of standard intravenous infusion of ocrelizumab and the pharmacokinetics in the case when 125 mg of ocrelizumab having the composition of Example 1 according to the present invention was subcutaneously injected at 1-month intervals were predicted and compared. As a result, the minimum concentration ($C_{trough}$) of the drug was predicted to be 2.895 mg/L, and the average concentration ($C_{average}$) of the drug was predicted to be 8.65 mg/L. Accordingly, it was found that the minimum concentration of the drug was maintained at 0.031 mg/L or higher when compared with the pharmacokinetics of standard treatment at 6-month intervals using the intravenous route, and the average concentration of the drug was reduced by about 25% compared to 11.51 mg/L, which was obtained for the standard intravenous infusion, but it was confirmed that the concentration was maintained similarly to the effective concentration of the drug estimated to be about 10 mg/L (FIG. 9). According to a clinical study of ocrelizumab in patients with multiple sclerosis and rheumatoid arthritis (Study # ACT2847g), when a total of 20 mg of ocrelizumab was intravenously administered by injecting 10 mg of ocrelizumab twice at 15-day intervals, immediate B cell depletion was observed. Assuming that the human blood volume was about 5 L, the blood concentration of ocrelizumab was expected to be 3 mg/L to 5 mg/L when 20 mg of ocrelizumab was intravenously administered, it can be found that the average concentration is a concentration sufficient to show B cell depletion even when the safety margin was assumed to be 100%.

In addition, based on the above absorption kinetics and the pharmacokinetic model of the clearance phase, the pharmacokinetics of standard intravenous infusion of ocrelizumab and the pharmacokinetics in the case when 250 mg of ocrelizumab having the composition according to the present invention was subcutaneously injected at 2-month intervals were predicted and compared. As a result, the minimum concentration ($C_{trough}$) of the drug was predicted to be 1.41 mg/L, and the average concentration ($C_{average}$) of the drug was predicted to be 9.52 mg/L. Accordingly, it was found that the minimum concentration of the drug was maintained at 0.031 mg/L or higher when compared with the pharmacokinetics of standard treatment at 6-month intervals using the intravenous route, and the average concentration of the drug was reduced by about 17% compared to 11.51 mg/L, which was obtained for the standard intravenous infusion, but it was confirmed that the concentration was maintained very close to the effective concentration of the drug estimated to be about 10 mg/L (FIG. 10).

In addition, based on the above absorption kinetics and the pharmacokinetic model of the clearance phase, the pharmacokinetics of standard intravenous infusion of ocrelizumab and the pharmacokinetics in the case when 375 mg of ocrelizumab having the composition according to the present invention was subcutaneously injected at 3-month intervals were predicted and compared. As a result, the minimum concentration ($C_{trough}$) of the drug was predicted to be 0.61 mg/L, and the average concentration ($C_{average}$) of the drug was predicted to be 10.19 mg/L. Accordingly, it was found that the minimum concentration of the drug was maintained at 0.031 mg/L or higher when compared with the pharmacokinetics of standard treatment at 6-month intervals using the intravenous route, and the average concentration of the drug was reduced by about 11% compared to 11.51 mg/L, which was obtained for the standard intravenous infusion, but it was confirmed that the concentration was maintained at about 10 mg/L or higher, which is the estimated effective concentration of the drug (FIG. 11).

In addition, based on the above absorption kinetics and the pharmacokinetic model of the clearance phase, the pharmacokinetics of standard intravenous infusion of ocrelizumab and the pharmacokinetics in the case when 500 mg of ocrelizumab having the composition according to the present invention was subcutaneously injected at 4-month intervals, except that the initial administration of 500 mg was carried out in a way that 250 mg of ocrelizumab was administered twice at 2-week intervals, were predicted and compared. As a result, the minimum concentration ($C_{trough}$) of the drug was predicted to be 0.24 mg/L, and the average concentration ($C_{average}$) of the drug was predicted to be 10.68 mg/L. Accordingly, it was found that the minimum concentration of the drug was maintained at 0.031 mg/L or higher when compared with the pharmacokinetics of standard treatment at 6-month intervals using the intravenous route, and the average concentration of the drug was reduced by about 7% compared to 11.51 mg/L, which was obtained for the standard intravenous infusion, but it was confirmed that the concentration was maintained at about 10 mg/L or higher, which is the estimated effective concentration of the drug (FIG. 12).

In addition, based on the above absorption kinetics and the pharmacokinetic model of the clearance phase, the pharmacokinetics of standard intravenous infusion of ocrelizumab and the pharmacokinetics in the case when 600 mg of ocrelizumab having the composition according to the present invention was subcutaneously injected at 5-month intervals, except that the initial administration of 600 mg was carried out in a way that 300 mg of ocrelizumab was administered twice at 2-week intervals, were predicted and compared. As a result, the minimum concentration ($C_{trough}$) of the drug was predicted to be 0.086 mg/L, and the average concentration ($C_{average}$) of the drug was predicted to be 10.57 mg/L. Accordingly, it was found that the minimum concentration of the drug was maintained at 0.031 mg/L or higher when compared with the pharmacokinetics of standard treatment at 6-month intervals using the intravenous route, and the average concentration of the drug was reduced by about 8% compared to 11.51 mg/L, which was obtained for the standard intravenous infusion, but it was confirmed that the concentration was maintained at about 10 mg/L or higher, which is the estimated effective concentration of the drug (FIG. 13).

In addition, based on the above absorption kinetics and the pharmacokinetic model of the clearance phase, the pharmacokinetics of standard intravenous infusion of ocrelizumab and the pharmacokinetics in the case when 750 mg of ocrelizumab having the composition according to the present invention was subcutaneously injected at 6-month intervals, except that the initial administration of 750 mg was carried out in a way that 375 mg of ocrelizumab was administered twice at 2-week intervals, were predicted and compared. As a result, the minimum concentration ($C_{trough}$) of the drug was predicted to be 0.24 mg/L, and the average concentration ($C_{average}$) of the drug was predicted to be 10.68 mg/L. Accordingly, it was found that the minimum concentration of the drug was maintained at 0.031 mg/L or higher when compared with the pharmacokinetics of standard treatment at 6-month intervals using the intravenous route, and the average concentration of the drug was reduced by about 7% compared to 11.51 mg/L, which was obtained for the standard intravenous infusion, but it was confirmed that the concentration was maintained at about 10 mg/L or higher, which is the estimated effective concentration of the drug (FIG. 14).

From the above experimental results, it was confirmed that the subcutaneous administration of the pharmaceutical composition including ocrelizumab and hyaluronidase according to the present invention can be provided as a safe therapeutic agent with significantly improved risk of side effects in intravenous administration, while exhibiting excellent therapeutic effects on multiple sclerosis.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

**Claims**

1. A pharmaceutical composition for preventing or treating multiple sclerosis, comprising ocrelizumab; and hyaluronidase,

   wherein, in the composition, ocrelizumab is subcutaneously administered to patients with multiple sclerosis at a dose of 40 mg to 1,200 mg.

2. The pharmaceutical composition of claim 1, wherein the pharmaceutical composition targets CD20.

3. The pharmaceutical composition of claim 1, wherein the hyaluronidase is selected from the group consisting of a combination of human or animal-derived PH20 and variants thereof.

4. The pharmaceutical composition of claim 3, wherein the hyaluronidase comprises an amino acid sequence of SEQ ID NO: 2.

5. The pharmaceutical composition of claim 1, wherein the ocrelizumab in the pharmaceutical composition is subcutaneously administered to patients with multiple sclerosis at a dose of 125 mg to 750 mg.

6. The pharmaceutical composition of claim 1, wherein the pharmaceutical composition comprises 10 mg/mL to 240 mg/mL of ocrelizumab.

7. The pharmaceutical composition of claim 1, wherein the pharmaceutical composition comprises 1,000 U/mL to 12,000 U/mL of hyaluronidase.

8. The pharmaceutical composition of claim 1, wherein the pharmaceutical composition further comprises a buffer at pH 5.5±2.0, a stabilizer, a nonionic surfactant, and a combination thereof.

9. The pharmaceutical composition of claim 1, wherein the pharmaceutical composition is administered once every 2 to 28 weeks.

10. The pharmaceutical composition of claim 1, wherein the pharmaceutical composition exhibits efficacy equal to or higher than that of intravenous administration as well as improves infusion related reactions (IRR).

11. A pharmaceutical composition for preventing or treating multiple sclerosis, comprising 250 mg to 750 mg of ocrelizumab; and hyaluronidase.

12. The pharmaceutical composition of claim 11, wherein the pharmaceutical composition is subcutaneously administered once or more every 4 to 28 weeks.

13. A pharmaceutical composition for preventing or treating multiple sclerosis, comprising 375 mg to 750 mg of ocrelizumab; and hyaluronidase.

14. The pharmaceutical composition of claim 13, wherein the pharmaceutical composition is subcutaneously administered once or more every 8 to 28 weeks.

15. A pharmaceutical composition for CD20 targeted therapy, comprising ocrelizumab; and hyaluronidase, wherein, in the composition, ocrelizumab is subcutaneously administered at a dose of 40 mg to 1,200 mg to patients targeted for CD20 targeted therapy.

16. A method for preventing or treating multiple sclerosis, comprising: subcutaneously administering 40 mg to 1,200 mg of ocrelizumab or a pharmaceutical composition comprising the same to a subject.

17. The method of claim 16, wherein the method further comprises administering hyaluronidase, and in particular, the hyaluronidase is administered simultaneously, sequentially, or in reverse order with ocrelizumab.

[FIG. 1]

[FIG. 2]

-◆-G1 (IV, 1 mg/kg)  -■-G2 (IV, 4 mg/kg)

-●-G3 (IV, 10 mg/kg)  -▲-G4 (IV, 20 mg/kg)

[FIG. 3]

-○-G5 (SC, 1 mg/kg)  -●-G6 (SC, 4 mg/kg)

-■-G7 (SC, 10 mg/kg)  -◆-G8 (SC, 20 mg/kg)

[FIG. 4]

EP 4 516 287 A1

[FIG. 5]

—O— G8 (SC, 20 mg/kg , with hyaluronidase )

···O··· G9 (SC, 20 mg/kg without hyaluronidase )

[FIG. 6]

[FIG. 7]

a. 400 mg intravenous injection
b. 1000 mg intravenous injection
c. 1500 mg intravenous injection
d. 2000 mg intravenous injection

[FIG. 8]

| Parameter | Reference |
|---|---|
| Cpeak,ss (mg/L) | 204.3 |
| Ctrough,ss (mg/L) | 0.031 |
| Partial AUC (day·mg/L) | 2071.77 |
| Caverage (mg/L) | 11.51 |

EP 4 516 287 A1

[FIG. 9]

| Parameter | Reference | Test6 |
|---|---|---|
| Cpeak,ss (mg/L) | 204.3 | 25.09 |
| Ctrough,ss (mg/L) | 0.031 | 2.895 |
| Partial AUC (day·mg/L) | 2071.77 | 259.423 |
| Caverage (mg/L) | 11.51 | 8.65 |

[FIG. 10]

| Parameter | Reference | Test5 |
|---|---|---|
| Cpeak,ss (mg/L) | 204.3 | 47.45 |
| Ctrough,ss (mg/L) | 0.031 | 1.41 |
| Partial AUC (day·mg/L) | 2071.77 | 571.451 |
| Caverage (mg/L) | 11.51 | 9.52 |

EP 4 516 287 A1

[FIG. 11]

| Parameter | Reference | Test4 |
|---|---|---|
| Cpeak,ss (mg/L) | 204.3 | 70.73 |
| Ctrough,ss (mg/L) | 0.031 | 0.61 |
| Partial AUC (day·mg/L) | 2071.77 | 917.43 |
| Caverage (mg/L) | 11.51 | 10.19 |

[FIG. 12]

| Parameter | Reference | Test3 |
|---|---|---|
| Cpeak,ss (mg/L) | 204.3 | 94.28 |
| Ctrough,ss (mg/L) | 0.031 | 0.24 |
| Partial AUC (day·mg/L) | 2071.77 | 1282.14 |
| Caverage (mg/L) | 11.51 | 10.68 |

[FIG. 13]

| Parameter | Reference | Test2 |
|---|---|---|
| Cpeak,ss (mg/L) | 204.3 | 113.2 |
| Ctrough,ss (mg/L) | 0.031 | 0.086 |
| Partial AUC (day·mg/L) | 2071.77 | 1584.87 |
| Caverage (mg/L) | 11.51 | 10.57 |

[FIG. 14]

| Parameter | Reference | Test1 |
|---|---|---|
| Cpeak,ss (mg/L) | 204.3 | 141.6 |
| Ctrough,ss (mg/L) | 0.031 | 0.034 |
| Partial AUC (day·mg/L) | 2071.77 | 2055.78 |
| Caverage (mg/L) | 11.51 | 11.42 |

## INTERNATIONAL SEARCH REPORT

| International application No. |
| --- |
| **PCT/KR2023/005185** |

**A. CLASSIFICATION OF SUBJECT MATTER**

**A61K 9/00**(2006.01)i; **A61K 47/42**(2006.01)i; **A61P 25/00**(2006.01)i; **A61P 25/28**(2006.01)i; **A61P 37/00**(2006.01)i; **C07K 16/28**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

A61K 9/00(2006.01); A61K 39/395(2006.01); A61K 47/18(2006.01); A61K 47/20(2006.01); A61K 47/42(2006.01); A61K 9/08(2006.01); C07K 16/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 오크렐리주맙(ocrelizumab), 히알루로니다제(hyaluronidase), 항체(antibody), 다발성 경화증(multiple sclerosis), 피하주사(subcutaneous injection)

**C. DOCUMENTS CONSIDERED TO BE RELEVANT**

| Category* | Citation of document, with indication, where appropriate, of the relevant passages | Relevant to claim No. |
| --- | --- | --- |
| X | KR 10-2020-0130451 A (ALTEOGEN, INC.) 18 November 2020 (2020-11-18)<br>See abstract; claims 1, 21-24, 30 and 33; paragraphs [0003], [0011], [0018], [0019], [0124], [0198], [0199] and [0207]; and SEQ ID NO: 1. | 1-15 |
| X | KR 10-2014-0006112 A (F. HOFFMANN-LA ROCHE AG) 15 January 2014 (2014-01-15)<br>See abstract; and claims 1-4. | 1 |
| A | WO 2009-009407 A1 (SMITHKLINE BEECHAM CO.) 15 January 2009 (2009-01-15)<br>See claims 1, 2 and 24. | 1-15 |
| A | KR 10-2020-0113292 A (GENENTECH, INC. et al.) 06 October 2020 (2020-10-06)<br>See claims 1 and 9. | 1-15 |
| A | KR 10-2013-0086628 A (GENENTECH, INC. et al.) 02 August 2013 (2013-08-02)<br>See claims 1 and 18. | 1-15 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **18 July 2023** | **18 July 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2023/005185** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/KR2023/005185**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **16, 17**
   because they relate to subject matter not required to be searched by this Authority, namely:

   Claims 16 and 17 pertain to a method for treatment of the human body, and thus pertain to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

<div align="center">

**INTERNATIONAL SEARCH REPORT**
**Information on patent family members**

</div>

| International application No. |
| --- |
| **PCT/KR2023/005185** |

| Patent document<br>cited in search report | | | Publication date<br>(day/month/year) | Patent family member(s) | | | Publication date<br>(day/month/year) |
| --- | --- | --- | --- | --- | --- | --- | --- |
| KR | 10-2020-0130451 | A | 18 November 2020 | AU | 2020-248612 | A1 | 21 October 2021 |
| | | | | CA | 3131052 | A1 | 01 October 2020 |
| | | | | CL | 2021002464 | A1 | 22 July 2022 |
| | | | | CN | 112203642 | A | 08 January 2021 |
| | | | | CO | 2021012380 | A2 | 20 October 2021 |
| | | | | CR | 20210489 | A | 07 December 2021 |
| | | | | DO | P2021000197 | A | 15 November 2021 |
| | | | | EA | 202192588 | A1 | 17 March 2022 |
| | | | | EC | SP21070640 | A | 18 November 2021 |
| | | | | EP | 3785701 | A1 | 03 March 2021 |
| | | | | JP | 2021-526542 | A | 07 October 2021 |
| | | | | JP | 2022-095925 | A | 28 June 2022 |
| | | | | JP | 7166478 | B2 | 07 November 2022 |
| | | | | JP | 7295150 | B2 | 20 June 2023 |
| | | | | KR | 10-2022-0075238 | A | 07 June 2022 |
| | | | | KR | 10-2023-0037691 | A | 16 March 2023 |
| | | | | KR | 10-2507853 | B1 | 10 March 2023 |
| | | | | NI | 202100085 | A | 06 December 2021 |
| | | | | PE | 20220283 | A1 | 25 February 2022 |
| | | | | SG | 11202110512 | A | 28 October 2021 |
| | | | | US | 2021-0363270 | A1 | 25 November 2021 |
| | | | | US | 2022-0289864 | A1 | 15 September 2022 |
| | | | | WO | 2020-197230 | A1 | 01 October 2020 |
| | | | | ZA | 202108027 | B | 21 December 2022 |
| KR | 10-2014-0006112 | A | 15 January 2014 | AR | 078161 | A1 | 19 October 2011 |
| | | | | AU | 2010-294186 | A1 | 08 March 2012 |
| | | | | AU | 2010-294186 | B2 | 19 September 2013 |
| | | | | BR | 112012005017 | A2 | 27 October 2020 |
| | | | | BR | 112012005017 | B1 | 08 September 2021 |
| | | | | CA | 2771571 | A1 | 17 March 2011 |
| | | | | CA | 2771571 | C | 12 November 2013 |
| | | | | CL | 2012000620 | A1 | 06 September 2013 |
| | | | | CN | 102686216 | A | 19 September 2012 |
| | | | | CN | 102686216 | B | 05 November 2014 |
| | | | | CN | 104399075 | A | 11 March 2015 |
| | | | | CN | 104399075 | B | 06 November 2018 |
| | | | | CO | 6440527 | A2 | 15 May 2012 |
| | | | | CR | 20120090 | A | 12 July 2012 |
| | | | | EP | 2475353 | A2 | 18 July 2012 |
| | | | | EP | 2475353 | B1 | 13 April 2016 |
| | | | | EP | 3061445 | A1 | 31 August 2016 |
| | | | | EP | 3064196 | A1 | 07 September 2016 |
| | | | | HK | 1208176 | A1 | 26 February 2016 |
| | | | | HR | P20160807 | T1 | 26 August 2016 |
| | | | | HU | E029078 | T2 | 28 February 2017 |
| | | | | JP | 2013-504540 | A | 07 February 2013 |
| | | | | JP | 2015-061839 | A | 02 April 2015 |
| | | | | JP | 2017-200924 | A | 09 November 2017 |
| | | | | JP | 2020-011962 | A | 23 January 2020 |
| | | | | JP | 5674788 | B2 | 25 February 2015 |

Form PCT/ISA/210 (patent family annex) (July 2022)

INTERNATIONAL SEARCH REPORT
Information on patent family members

International application No.

**PCT/KR2023/005185**

| Patent document cited in search report | | | Publication date (day/month/year) | Patent family member(s) | | | Publication date (day/month/year) |
|---|---|---|---|---|---|---|---|
| | | | | JP | 6364310 | B2 | 25 July 2018 |
| | | | | JP | 6934919 | B2 | 15 September 2021 |
| | | | | KR | 10-1782195 | B1 | 26 September 2017 |
| | | | | KR | 10-1782301 | B1 | 26 September 2017 |
| | | | | KR | 10-2012-0051094 | A | 21 May 2012 |
| | | | | KR | 10-2017-0034441 | A | 28 March 2017 |
| | | | | MA | 33592 | B1 | 01 September 2012 |
| | | | | MX | 2012002861 | A | 20 April 2012 |
| | | | | MX | 360432 | B | 31 October 2018 |
| | | | | MX | 360435 | B | 31 October 2018 |
| | | | | MY | 174009 | A | 03 March 2020 |
| | | | | US | 10280227 | B2 | 07 May 2019 |
| | | | | US | 10377831 | B2 | 13 August 2019 |
| | | | | US | 10752696 | B2 | 25 August 2020 |
| | | | | US | 2011-0076273 | A1 | 31 March 2011 |
| | | | | US | 2015-0086537 | A1 | 26 March 2015 |
| | | | | US | 2016-0137742 | A1 | 19 May 2016 |
| | | | | US | 2019-0367629 | A1 | 05 December 2019 |
| | | | | US | 2021-0054092 | A1 | 25 February 2021 |
| | | | | WO | 2011-029892 | A2 | 17 March 2011 |
| | | | | WO | 2011-029892 | A3 | 15 September 2011 |
| | | | | ZA | 201201605 | B | 25 September 2019 |
| WO | 2009-009407 | A1 | 15 January 2009 | AR | 067455 | A1 | 14 October 2009 |
| | | | | AR | 109461 | A2 | 12 December 2018 |
| | | | | AU | 2008-275278 | A1 | 15 January 2009 |
| | | | | AU | 2008-275278 | B2 | 14 April 2011 |
| | | | | BR | PI0814003 | A2 | 03 February 2015 |
| | | | | BR | PI0814003 | B1 | 15 June 2021 |
| | | | | CA | 2692681 | A1 | 15 January 2009 |
| | | | | CA | 2692681 | C | 04 December 2018 |
| | | | | CL | 2008001984 | A1 | 09 January 2009 |
| | | | | CN | 101820912 | A | 01 September 2010 |
| | | | | CN | 101820912 | B | 12 March 2014 |
| | | | | EP | 2170388 | A1 | 07 April 2010 |
| | | | | EP | 2170388 | B1 | 12 October 2016 |
| | | | | EP | 2889310 | A1 | 01 July 2015 |
| | | | | EP | 2889310 | B1 | 20 December 2017 |
| | | | | JP | 2011-526880 | A | 20 October 2011 |
| | | | | JP | 5529017 | B2 | 25 June 2014 |
| | | | | KR | 10-1670454 | B1 | 31 October 2016 |
| | | | | KR | 10-2010-0038092 | A | 12 April 2010 |
| | | | | KR | 10-2015-0088335 | A | 31 July 2015 |
| | | | | US | 2011-0020328 | A1 | 27 January 2011 |
| | | | | US | 2015-0158951 | A1 | 11 June 2015 |
| | | | | UY | 31210 | A1 | 30 January 2009 |
| | | | | ZA | 200909107 | B | 27 December 2012 |
| KR | 10-2020-0113292 | A | 06 October 2020 | AU | 2018-210312 | A1 | 04 July 2019 |
| | | | | AU | 2018-210312 | B2 | 19 March 2020 |
| | | | | AU | 2020-203066 | A1 | 28 May 2020 |
| | | | | CA | 3047349 | A1 | 26 July 2018 |

Form PCT/ISA/210 (patent family annex) (July 2022)

EP 4 516 287 A1

**INTERNATIONAL SEARCH REPORT**
**Information on patent family members**

| International application No. |
| --- |
| **PCT/KR2023/005185** |

| Patent document cited in search report | | | Publication date (day/month/year) | Patent family member(s) | | | Publication date (day/month/year) |
| --- | --- | --- | --- | --- | --- | --- | --- |
| | | | | CA | 3047349 | C | 22 September 2020 |
| | | | | CN | 110167594 | A | 23 August 2019 |
| | | | | CN | 111714630 | A | 29 September 2020 |
| | | | | CN | 111714630 | B | 09 November 2021 |
| | | | | CN | 115089704 | A | 23 September 2022 |
| | | | | EP | 3570884 | A2 | 27 November 2019 |
| | | | | EP | 3570884 | B1 | 30 September 2020 |
| | | | | EP | 3868404 | A1 | 25 August 2021 |
| | | | | JP | 2020-186239 | A | 19 November 2020 |
| | | | | JP | 2020-515519 | A | 28 May 2020 |
| | | | | JP | 2021-006526 | A | 21 January 2021 |
| | | | | JP | 6741875 | B2 | 19 August 2020 |
| | | | | JP | 6769687 | B1 | 14 October 2020 |
| | | | | KR | 10-2019-0104554 | A | 10 September 2019 |
| | | | | KR | 10-2021-0134432 | A | 09 November 2021 |
| | | | | KR | 10-2022-0162863 | A | 08 December 2022 |
| | | | | US | 10849849 | B2 | 01 December 2020 |
| | | | | US | 11654105 | B2 | 23 May 2023 |
| | | | | US | 2018-0296470 | A1 | 18 October 2018 |
| | | | | US | 2021-0085597 | A1 | 25 March 2021 |
| | | | | US | 2023-0092354 | A1 | 23 March 2023 |
| | | | | WO | 2018-136412 | A2 | 26 July 2018 |
| | | | | WO | 2018-136412 | A3 | 15 November 2018 |
| KR | 10-2013-0086628 | A | 02 August 2013 | BR | 112013011305 | A2 | 25 July 2017 |
| | | | | CA | 2817216 | A1 | 18 May 2012 |
| | | | | CN | 103476793 | A | 25 December 2013 |
| | | | | CN | 104998254 | A | 28 October 2015 |
| | | | | EP | 2638067 | A2 | 18 September 2013 |
| | | | | EP | 2787007 | A2 | 08 October 2014 |
| | | | | EP | 2787007 | A3 | 11 March 2015 |
| | | | | EP | 3351559 | A2 | 25 July 2018 |
| | | | | EP | 3351559 | A3 | 31 October 2018 |
| | | | | EP | 4029881 | A1 | 20 July 2022 |
| | | | | JP | 2013-541594 | A | 14 November 2013 |
| | | | | JP | 2015-134765 | A | 27 July 2015 |
| | | | | JP | 2017-081937 | A | 18 May 2017 |
| | | | | JP | 2019-112420 | A | 11 July 2019 |
| | | | | JP | 2021-050215 | A | 01 April 2021 |
| | | | | JP | 2023-036669 | A | 14 March 2023 |
| | | | | JP | 6486887 | B2 | 20 March 2019 |
| | | | | JP | 6803937 | B2 | 23 December 2020 |
| | | | | KR | 10-1933197 | B1 | 27 December 2018 |
| | | | | KR | 10-2015-0055107 | A | 20 May 2015 |
| | | | | KR | 10-2019-0120439 | A | 23 October 2019 |
| | | | | KR | 10-2020-0059320 | A | 28 May 2020 |
| | | | | KR | 10-2021-0021109 | A | 24 February 2021 |
| | | | | KR | 10-2022-0070586 | A | 31 May 2022 |
| | | | | KR | 10-2116202 | B1 | 28 May 2020 |
| | | | | US | 10231981 | B2 | 19 March 2019 |
| | | | | US | 10874677 | B2 | 29 December 2020 |

Form PCT/ISA/210 (patent family annex) (July 2022)

**INTERNATIONAL SEARCH REPORT**
Information on patent family members

International application No.

**PCT/KR2023/005185**

| Patent document cited in search report | Publication date (day/month/year) | Patent family member(s) | | | Publication date (day/month/year) |
|---|---|---|---|---|---|
| | | US | 11622969 | B2 | 11 April 2023 |
| | | US | 2012-0301460 | A1 | 29 November 2012 |
| | | US | 2014-0056883 | A1 | 27 February 2014 |
| | | US | 2014-0056884 | A1 | 27 February 2014 |
| | | US | 2014-0056885 | A1 | 27 February 2014 |
| | | US | 2017-0360807 | A1 | 21 December 2017 |
| | | US | 2019-0247403 | A1 | 15 August 2019 |
| | | US | 2021-0228597 | A1 | 29 July 2021 |
| | | US | 2022-0280532 | A1 | 08 September 2022 |
| | | US | 8580264 | B2 | 12 November 2013 |
| | | US | 9539263 | B2 | 10 January 2017 |
| | | US | 9750752 | B2 | 05 September 2017 |
| | | WO | 2012-064627 | A2 | 18 May 2012 |
| | | WO | 2012-064627 | A3 | 19 July 2012 |

Form PCT/ISA/210 (patent family annex) (July 2022)

# EP 4 516 287 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20130099228 A
- KR 102151388
- WO 761053 A

### Non-patent literature cited in the description

- **EDWARDS et al.** *Biochem Scoc. Trans.*, 2002, vol. 30, 824-828
- **PEARSON et al.** *Proc. Natl. Acad. Sci. USA*, 1988, vol. 85, 2444
- **NEEDLEMAN ; WUNSCH.** *J. Mol. Biol.*, 1970, vol. 48, 443-453
- **RICE et al.** EMBOSS: The European Molecular Biology Open Software Suite. *Trends Genet.*, 2000, vol. 16, 276-277
- **DEVEREUX, J. et al.** *Nucleic Acids Research*, 1984, vol. 12, 387
- **ATSCHUL, S. F. et al.** *J MOLEC BIOL*, 1990, vol. 215, 403
- **MARTIN J. BISHOP.** Guide to Huge Computers. Academic Press, 1994
- **CARILLO et al.** *SIAM J Applied Math*, 1988, vol. 48, 1073
- **NEEDLEMAN et al.** *J Mol Biol.*, 1970, vol. 48, 443
- **SMITH ; WATERMAN.** *Adv. Appl. Math*, 1981, vol. 2, 482
- **GRIBSKOV et al.** *Nucl. Acids Res.*, 1986, vol. 14, 6745
- Atlas of Protein Sequence and Structure. National Biomedical Research Foundation, 1979, 353-358